# EUROPEAN PATENT SPECIFICATION

(11) **EP 3 245 219 B1**
(45) Date of publication and mention of the grant of the patent: **08.04.2020**
(21) Application number: 15877381.2
(22) Date of filing: 16.01.2015
(51) Int. Cl.: C07K 7/06, C07K 7/08, C07K 14/705, C07K 16/28, G01N 33/53

(54) **GLYPICAN EPITOPES AND USES THEREOF**
GLYPICANEPITOPE UND VERWENDUNGEN DAVON
ÉPITOPES DE LA GLYPICANE ET LEURS UTILISATIONS

(43) Date of publication of application: 22.11.2017
(73) Proprietor: GlyP Holdings Pty Limited, Macquarie Park NSW 2113 (AU)
(72) Inventor: CAMPBELL, Douglas, Cremorne NSW 2090 (AU); JUSTINIANO FUENMAYOR, Irene, St Ives, NSW 2075 (AU); NOCON, Aline, Leichardt, NSW 2040 (AU); SOON, Julie, North Bondi, NSW 2026 (AU); TRUONG, Quach, Leichardt, NSW 2040 (AU); WALSH, Bradley, East Lindfield NSW 2070 (AU); WISSMUELLER, Sandra, Currans Hill, NSW 2567 (AU)
(74) Representative: Novitas Patent AB
(86) International application number: PCT/AU2015/000019
(87) International publication number: WO 2016/112423

(56) References cited:
- WO-A1-00/23109
- WO-A2-99/37764
- US-A1- 2004 226 056
- DATABASE PROTEIN [Online] 01 February 1994 XP055455319 Retrieved from NCBI Database accession no. P35052
- 'Anti-GPC antibody HPA030571' 26 March 2010, XP055461315 Retrieved from the Internet: <URL:http://www.proteinatlas.org/ENSG000000 63660-GPC1/antibody> [retrieved on 2015-02-11]
- SUHOVSKIH, A. ET AL.: 'Proteoglycan Expression in Normal Human Prostate Tissue and Prostate Cancer.' ISRN ONCOLOGY vol. 2013, 2013, page 680136, XP055357331

## Description

### Technical Field

The present invention relates generally to the fields of immunology and medicine. More specifically, the present invention relates to epitopes of glypican-1 (GPC-1) and uses thereof.

### Background

Prostate cancer is the most commonly occurring cancer in men of all races and is second only to lung cancer in mortality among among white, black, American Indian/Alaska Native, and Hispanic men. In 2011, 209,292 men in the United States of America were diagnosed with prostate cancer and 27,970 of these died from the disease (U.S. Cancer Statistics Working Group, "United States Cancer Statistics: 1999-2011 Incidence and Mortality Web-based Report", Atlanta (GA): Department of Health and Human Services, Centers for Disease Control and Prevention, and National Cancer Institute; 2014).

Treatment with surgery and/or radiotherapy is successful in many patients if prostate cancer is diagnosed early. However, many patients with advanced disease and a sizeable proportion of all prostate cancer patients eventually develop metastatic disease following localised therapy.

A need thus exists for convenient, reliable and accurate tests for diagnosing prostate cancer, especially during the early stages of the disease.

Glypican-1 (GPC-1) is a cell surface heparan sulfate proteoglycan with a core protein that is anchored to the cytoplasmic membrane via a glycosyl phosphatidylinositol. It is a member of a larger family of glypicans. The sequence of GPC-1 and the encoding nucleic acid has been disclosed in e.g. WO 99/37764. GPC-1 has been reported to be overexpressed in some forms of cancer (e.g. pancreatic cancer, breast cancer), but expression does not significantly differ in others. The present inventors have recently determined that GPC-1 is overexpressed by prostate cancer cells, and can be used as a means of diagnosing the disease (US provisional patent application no. 61/928/776 entitled "*Cell Surface Prostate Cancer Antigen for Diagnosis*", Walsh *et al.* - unpublished; PCT application number PCT/AU2014/000999 *"Monoclonal ANTI-GPC-1 antibodies and uses thereof",* Walsh *et al.* - unpublished).

In view of the association between GPC-1 levels and prostate cancer determined by the present inventors, a need exists for the identification of epitopes within GPC-1 that are advantageous for detecting and quantifying GPC-1 levels in persons undergoing diagnostic and/or prognostic tests.

### Summary of the Invention

The present inventors have determined that a series of epitopes within the GPC-1 protein are preferably targeted by binding entities including, but not limited to, antibodies. The patent relates to the invention as it is defined in the appended claims.

Disclosed herein are the following embodiments:
Embodiment 1: An epitope for an anti-glypican 1 (GPC-1) antibody located within a portion of the GPC-1 flexible loop defined by an amino acid sequence KVNPQGPGPEEK (**SEQ ID NO: 1**).
Embodiment 2: The epitope according to embodiment 1, wherein the epitope comprises a first segment comprising an amino acid sequence selected from:
   (i) VNPQGPGPEEK (**SEQ ID NO: 2**); or
   (ii) a variant of VNPQGPGPEEK (**SEQ ID NO: 2**), wherein the variant comprises a substituted amino acid residue only at:
      any one or more of positions 2, 3, 7, 8, 9, 10 and/or 11; or
      any one or more of positions 1, 2, 3, 4, 6, 8, 10 and/or 11; or
      any one or more of positions 1, 2, 3, 4, 5, 8, 10 and/or 11.
Embodiment 3: The epitope according to embodiment 1 wherein the epitope comprises a first segment comprising an amino acid sequence selected from:
   (i) KVNPQGPGP (**SEQ ID NO: 6**); or
   (ii) a variant of KVNPQGPGP (**SEQ ID NO: 6**) comprising a substituted amino acid residue only at any one or more of positions 1, 3, 4, 8, and 9 of **SEQ ID NO: 6.**
Embodiment 4: The variant according to embodiment 3, comprising a substituted amino acid residue only at any one or more of positions 8 and 9 of **SEQ ID NO: 6.**
Embodiment 5: The epitope according to embodiment 1, wherein the epitope comprises a first segment comprising an amino acid sequence selected from:
   (i) KVNPQGPGPE (**SEQ ID NO: 5**); or
   (ii) a variant of KVNPQGPGPE (**SEQ ID NO: 5**) comprising a substituted amino acid residue only at any one or more of positions 1, 3, 4, 8, 9 and 10 of **SEQ ID NO: 5.**
Embodiment 6: The variant according to embodiment 5, comprising a substituted amino acid residue only at any one or more of positions 8, 9 and 10 of **SEQ ID NO: 6.**
Embodiment 7: The variant according to embodiment 5 or embodiment 6, wherein E (glu) at position 10 is substituted with any other amino acid.
Embodiment 8: The variant according to any one of embodiments 3 to 7, wherein the variant comprises a substitution only at any one or more of:
   position 1, wherein K (lys) is substituted with any one of W (trp), R (arg), L (lys), Y (tyr) or F (phe);
   position 3, wherein N (asn) is substituted with any one of H (his), P (pro) or D (asp);
   position 4, wherein P (pro) is substituted with any one of R (arg), K (lys), W (trp), S (ser), H (his) or N (asn);
   position 8, wherein G (gly) is substituted with any one of D (asp), E (glu), N (asn), Q (gln), K (lys), R (arg) or A (ala);
   position 9, wherein P (pro) is substituted with any one of M (met), A (ala), I (ile), K (lys), R (arg), Q (gln), S (ser), T (thr), or Y (tyr).
Embodiment 9: The epitope according to any one of embodiments 1 to 8, comprising a second segment comprising an amino acid sequence TQNARA (**SEQ ID NO: 8**).
Embodiment 10: The epitope according to any one of embodiments 1 to 8, comprising a second segment comprising an amino acid sequence TQNARAFRD (**SEQ ID NO: 7**).
Embodiment 11: The epitope according to embodiment 1 or embodiment 2, wherein the epitope comprises a first segment comprising an amino acid sequence selected from:
   (i) NPQGPGPEE (**SEQ ID NO: 4**); or
   (ii) a variant of NPQGPGPEE (**SEQ ID NO: 4**), wherein the variant comprises a substituted amino acid residue only at any one or more of positions 1, 2, 3, 5, 7 and 9 of **SEQ ID NO: 4.**
Embodiment 12: The variant according to embodiment 11, comprising a substituted amino acid residue only at any one or more of positions 2, 7, and 9 of **SEQ ID NO: 4.**
Embodiment 13: The variant according to embodiment 11 or embodiment 12, wherein the variant comprises a substitution at any one or more of:
   position 1, wherein N (asn) is substituted with H (his);
   position 2, wherein P (pro) is substituted with any other amino acid;
   position 3, wherein Q (gln) is substituted with any one of N (asn), M (met), T (thr), S (ser), or R (arg);
   position 5, wherein P (pro) is substituted with A (ala);
   position 7, wherein P (pro) is substituted with any one of A (ala), D (asp), C (cys), E (glu), Z (glx), G (gly), H (his), K (lys), M (met), F (phe), P (pro), S (ser), T (thr), W (trp), or Y (tyr);
   position 9, wherein E (glu) is substituted with any other amino acid.
Embodiment 14: The epitope according to any one of embodiments 11 to 13, wherein the epitope comprises a second segment comprising an amino acid sequence ALSTASDDR (**SEQ ID NO: 9**).
Embodiment 15: The epitope according to embodiment 1 or embodiment 2, wherein the epitope comprises a first segment comprising an amino acid sequence selected from:
   (i) VNPQGPGPEE (**SEQ ID NO: 3**); or
   (ii) a variant of VNPQGPGPEE (**SEQ ID NO: 3**), wherein the variant comprises a substituted amino acid residue only at any one or more of positions 1, 2, 3, 4, 5, 8 and 10 of **SEQ ID NO: 3.**
Embodiment 16: The variant according to embodiment 15, comprising a substituted amino acid residue only at any one or more of positions 1, 2, 3, and 8 of **SEQ ID NO: 3.**
Embodiment 17: The variant according to embodiment 15 or embodiment 16, wherein the variant comprises a substitution at any one or more of:
   position 1, wherein V (val) is substituted with any other amino acid;
   position 2, wherein N (asn) is substituted with any other amino acid;
   position 3, wherein P (pro) is substituted with any other amino acid;
   position 4, wherein Q (gln) is substituted with any one of Y (tyr), A (ala), E (glu), V (val), M (met), F (phe), L (leu), I (ile), T (thr), or R (arg);
   position 5, wherein G (gly) is substituted with A (ala), S (ser), T (thr), H (his), W (trp), Y (tyr), F (phe), or M (met);
   position 8, wherein P (pro) is substituted with any other amino acid;
   position 10, wherein E (glu) is substituted with Q (gln), D (asp), F (phe), H (his) or M (met).
Embodiment 18: The epitope according to any one of embodiments 15 to 17, wherein the epitope comprises a second segment comprising:
   an amino acid sequence PRERPP (**SEQ ID NO: 10**) or
   an amino acid sequence QDASDDGSGS (**SEQ ID NO: 11**).
Embodiment 19: The epitope according to any one of embodiments 15 to 17, wherein the epitope comprises a second segment comprising an amino acid sequence PRERPP (**SEQ ID NO: 10**) and a third segment comprising an amino acid sequence QDASDDGSGS (**SEQ ID NO: 11**).
Embodiment 20: The epitope according to any one of embodiments 1 to 10, comprising the amino acid sequence CGELYTQNARAFRDLCGNPKVNPQGPGPEEKRRRGC (**SEQ ID NO: 12**).
Embodiment 21: The epitope according to any one of embodiments 1 to 20, wherein the epitope is a linear epitope.
Embodiment 22: The epitope according to embodiment 19, wherein the second segment and the third segment of the epitope are discontinuous.
Embodiment 23: The epitope according to any one of embodiments 9, 10, 14, 18 or 22 wherein the first segment and the second segment of the epitope are discontinuous.
Embodiment 24: An epitope for an anti-glypican 1 (GPC-1) antibody comprising an amino acid sequence selected from any one or a plurality of: TQNARA (**SEQ ID NO: 8**), ALSTASDDR (**SEQ ID NO: 9**), PRERPP (**SEQ ID NO: 10**), QDASDDGSGS (**SEQ ID NO: 11**), LGPECSRAVMK (**SEQ ID NO: 13**), and TQNARAFRD (**SEQ ID NO: 7**).
Embodiment 25: The epitope according to any one of embodiments 1 to 24, wherein the epitope is an isolated polypeptide or a synthetic polypeptide.
Embodiment 26: An arrangement of epitopes comprising a combination of two or more distinct epitopes, wherein
   each said distinct epitope is an epitope according to any one of embodiments 1 to 25;
   each said epitope is an epitope according to **Table 1**; or
   the combination of epitopes is any one or more of the combinations set out in **Table 3.**
Embodiment 27: An arrangement of epitopes comprising a combination of two or more distinct epitopes, wherein the arrangement comprises
   a first epitope according to any one of embodiments 1 to 10, and
   a second epitope according to any one of embodiments 11 to 14.
Embodiment 28: An arrangement of epitopes comprising a combination of two or more distinct epitopes, wherein the arrangement comprises
   a first epitope according to any one of embodiments 1 to 10, and
   a second epitope according to any one of embodiments 15 to 19.
Embodiment 29: A composition comprising an epitope according to any one of embodiments 1 to 25, or an arrangement of epitopes according to any one of embodiments 26 to 28, and a pharmaceutically acceptable carrier or excipient.
Embodiment 30: An assembly comprising an epitope according to any one of embodiments 1 to 25, or an arrangement of epitopes according to any one of embodiments 26 to 28, bound to one or more soluble or insoluble supports.
Embodiment 31: The assembly of embodiment 30, wherein the assembly is a component of an enzyme-linked immunosorbent assay (ELISA).
Embodiment 32: A nucleic acid encoding the epitope according to any one of embodiments 1 to 25.
Embodiment 33: A vector comprising the nucleic acid according to embodiment 32.
Embodiment 34: A host cell comprising the vector according to embodiment 33.
Embodiment 35: An isolated binding entity capable of specifically binding to an epitope according to any one of embodiments 1 to 25, wherein the binding entity is not an antibody.
Embodiment 36: An isolated binding entity capable of specifically binding to an epitope according to any one of embodiments 1 to 25, wherein the binding entity is an antibody and with the proviso that the antibody is not a: MIL38 antibody (CBA20140026), rabbit anti-GPC-1 polyclonal antibody (ab137604, abcam), mouse anti-glypican monoclonal antibody 2600 clone 4D1 (Millipore), or goat anti-glypican 1 antibody (AA 24-530).
Embodiment 37: A method for detecting prostate cancer in a subject, the method comprising obtaining a biological sample from the subject, detecting the presence of an epitope according to any one of embodiments 1 to 25 in the sample, and determining that the subject has prostate cancer or an increased likelihood of developing prostate cancer based on amount of the epitope detected in the sample.
   The biological sample may be a body fluid sample.
   The biological sample may be a tissue sample.
Embodiment 38: The method according to embodiment 37, wherein detecting the presence of the epitope in the sample comprises contacting the sample with a binding entity capable of specifically binding to an epitope according to any one of embodiments 1 to 25.
Embodiment 39: The method according to embodiment 38, wherein the binding entity is a population of antibodies.
Embodiment 40: The method according to embodiment 39, wherein the population of antibodies comprises any one or more of: MIL38 antibody (CBA20140026), rabbit anti-GPC-1 polyclonal antibody (ab137604, abcam), mouse anti-glypican monoclonal antibody 2600 clone 4D1 (Millipore), or goat anti-glypican 1 antibody (AA 24-530).
Embodiment 41: The method according to embodiment 39, wherein the population of antibodies does not contain any of: MIL38 antibody (CBA20140026), rabbit anti-GPC-1 polyclonal antibody (ab137604, abcam), mouse anti-glypican monoclonal antibody 2600 clone 4D1 (Millipore), or goat anti-glypican 1 antibody (AA 24-530).
Embodiment 42: The method according to any one of embodiments 37 to 41, comprising comparing the amount of epitope present in the biological sample with an amount of epitope present in a control sample, wherein the detection of an increased amount of epitope in the body fluid sample compared to an equivalent measure of the control sample is indicative of prostate cancer in the subject or an increased likelihood of developing prostate cancer in the subject.
Embodiment 43: The method according to embodiment 42, wherein the amount of epitope detected in the sample is increased by more than 50% over the amount of epitope detected in the control sample.
Embodiment 44: The method according to any one of embodiments 37 to 43, wherein detecting the presence of the epitope comprises contacting the sample with a population of MIL38 antibodies as deposited at Cellbank Australia under accession number CBA20140026.
Embodiment 45: The method according to any one of embodiments 37 to 43, wherein detecting the presence of the epitope comprises contacting the sample with a population of antibodies that does not comprise an antibody comprising a light chain variable region comprising: a complementarity determining region 1 (CDR1) comprising or consisting of an amino acid sequence defined by positions 48-58 of **SEQ ID NO: 20**; a complementarity determining region 2 (CDR2) comprising or consisting of an amino acid sequence defined by positions 74-80 of **SEQ ID NO: 20**; and/or a complementarity determining region 3 (CDR3) comprising or consisting of an amino acid sequence defined by positions 113-121 of **SEQ ID NO: 20.**
Embodiment 46: The method according to any one of embodiments 37 to 45, further comprising determining the level of prostate-specific antigen (PSA) in the biological sample and comparing the level detected to that of the control sample.
Embodiment 47: The method according to any one of embodiments 37 to 46, wherein the biological sample is a body fluid sample.
Embodiment 48: The method according to any one of embodiments 37 to 46, wherein the biological sample is a tissue sample.
Embodiment 49: A fusion protein comprising the epitope according to any one of embodiments 1 to 25.
Embodiment 50: Use of the epitope according to any one of embodiments 1 to 25, the arrangement of epitopes according to any one of embodiments 26 to 28, or the fusion protein according to embodiment 48, as a positive control element in a method for detecting GPC-1.
Embodiment 51: The use according to embodiment 50, wherein the method is the method for detecting prostate cancer according to any one of embodiments 37 to 48.

The positive control element when used in a detection method or detection assay, may directly or indirectly provide a positive/affirmative signal, and thereby at least in part or wholly validate that the method or assay is capable of functioning correctly.

### Brief Description of the Figures

Preferred embodiments of the present invention will now be described, by way of example only, with reference to the accompanying figures wherein:
**Figure 1** shows a rendering of chain B as present in Protein Data Bank identifier (PBD ID) 4AD7 (http://www.ebi.ac.uk/pdbsum/4AD7);
**Figure 2** shows box plot graphs of raw data of MIL38-AM4 antibody screening. The bottom and top of the boxes are the 25th and 75th percentile of the data. The band near the middle of the box is the 50th percentile (the median). The whiskers are at 1.5 the inter-quantile range, an indication of statistical outliers within the dataset (Mcgill et al., The American Statistician, 32: 12-16, 1978);
**Figure 3** shows plots of the peptide number versus the recorded intensity obtained for all five of arrays (sets 1-5, Example 1), when incubated with 1µg/ml (top trace) or 10 µg/ml of MIL38-AM4 (bottom trace);
**Figure 4** shows rendering of chain B from Protein Data Bank identifier (PBD ID) 4AD7 (http://www.ebi.ac.uk/pdbsum/4AD7) with sequence TQNARAFRDLYS (**SEQ ID NO: 21**) in dark-coloured spheres, and residues K347, V348, G362, and K363 in light-coloured spheres. The loop connecting V348 to G362 is not resolved from the X- ray diffraction;
**Figure 5** shows box plot graphs of raw data of polyclonal and monoclonal anti-GPC-1 and MIL38-AM3 antibody screening. The bottom and top of the boxes are the 25th and 75th percentile of the data. The band near the middle of the box is the 50th percentile (the median). The whiskers are at 1.5 the inter-quantile range, an indication of statistical outliers within the dataset (Mcgill et al., The American Statistician, 32: 12-16, 1978);
**Figure 6** shows plots of the peptide number versus the recorded intensity obtained for three commercially available anti-glypican 1 preparations tested on the array used to map MIL38-AM4, which is shown for comparison;

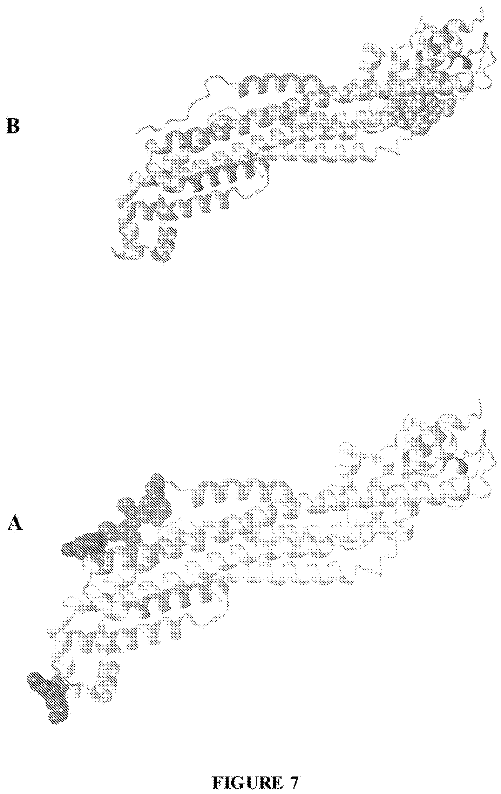

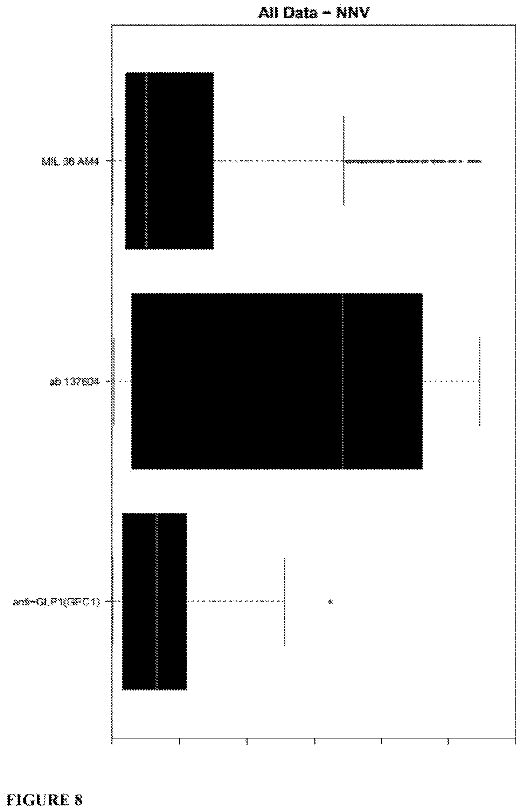

**Figure 7** shows rendering of chain B from Protein Data Bank identifier (PBD ID) 4AD7 (http://www.ebi.ac.uk/pdbsum/4AD7). **Figure 7A** shows rendering of chain B with sequence PRERPP (**SEQ ID NO: 10**) in light-shaded spheres, and residues K347, V348, G362, and K363 in dark-shaded spheres. The loop connecting V348 to G362 is not resolved from the X-ray diffraction. These epitopes are recognized by rabbit anti-GPC-1. An extra epitope, QDASDDGSGS (**SEQ ID NO: 11**), is not resolved in the coordinate file. **Figure 7B** shows rendering of chain B with sequence LGPECSRAVMK (**SEQ ID NO: 13**) in shaded spheres. This epitope is recognized by mouse anti-GPC-1; **Figure 8** shows box plot graphs of raw data of antibody screening. The bottom and top of the boxes are the 25th and 75th percentile of the data. The band near the middle of the box is the 50th percentile (the median). The whiskers are at 1.5 the inter-quantile range, an indication of statistical outliers within the dataset (Mcgill et al., The American Statistician, 32: 12-16, 1978);
**Figure 9** shows a letterplot representation of rabbit polyclonal Ab 137604 probed on the substitution analysis of set 3. The central/horizontal bar represents the mean intensity recorded for the base sequence. Individual mutations are drawn in the column corresponding to the position of the mutation, and symbols are plotted at the height corresponding to the recorded intensity;
**Figure 10** shows a letterplot representation of goat polyclonal anti-GPC-1 probed on the substitution analysis of set 2 (Example 3);

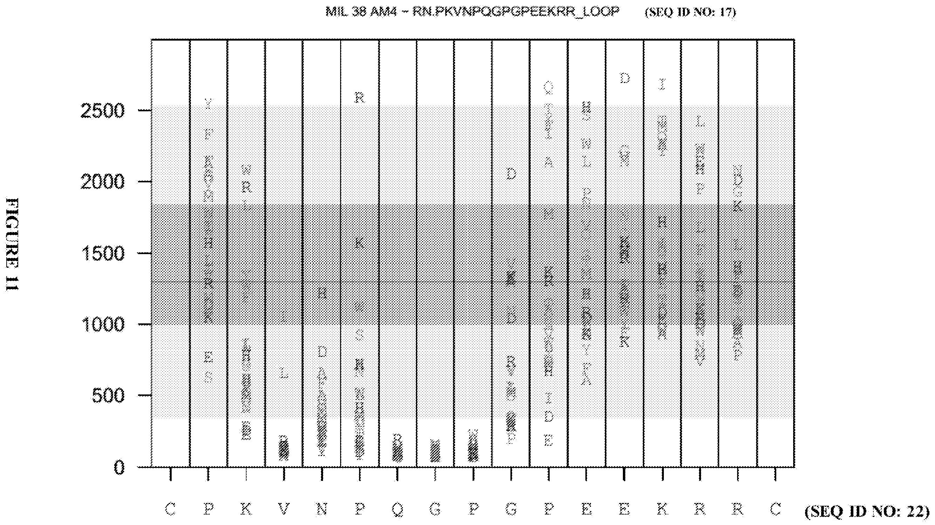

**Figure 11** shows a letterplot representation of MIL38-AM4 probed on the substitution analysis of set 3 (Example 3);

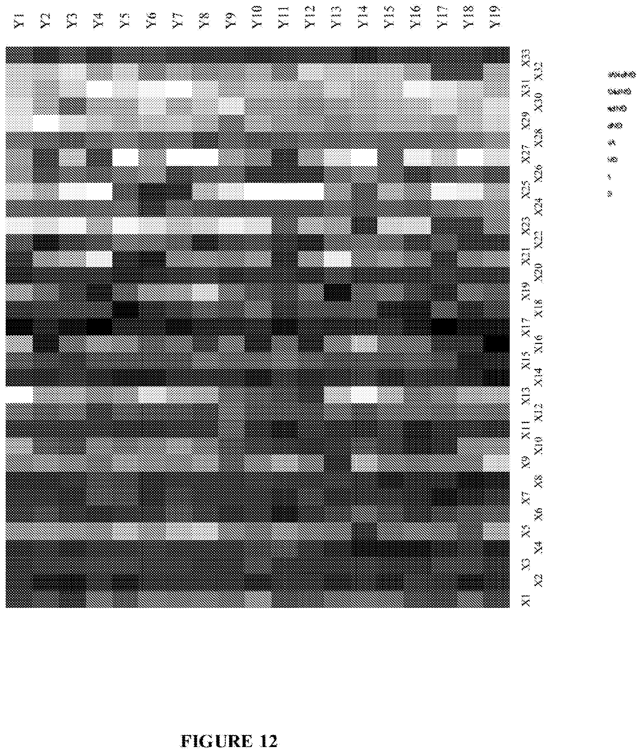

**Figure 12** shows a heatmap representation of the data obtained for MIL38-AM4 probed on the peptides of set 1 (Example 3). Signal intensity runs from black (baseline) to white (mean) to grey (max); 'X' = x-axis; x-axis peptide sequences depicted in columns X1-X33 are as follows: X-1 peptide is residues 344 - 353 of **SEQ ID NO: 1**; X-2 peptide is residues 348 - 357 of **SEQ ID NO: 1**; X-3 peptide is residues 352 - 361 of **SEQ ID NO: 1**; X-4 peptide is residues 356 - 365 of **SEQ ID NO: 1**; X-5 peptide is residues 344 - 354 of **SEQ ID NO: 1**; X-6 peptide is residues 348 - 358 of **SEQ ID NO: 1**; X-7 peptide is residues 352 - 362 of **SEQ ID NO: 1**; X-8 peptide is residues 356 - 366 of **SEQ ID NO: 1**; X-9 peptide is residues 344 - 355 of **SEQ ID NO: 1**; X-10 peptide is residues 348 - 359 of **SEQ ID NO: 1**; X-11 peptide is residues 352 - 363 of **SEQ ID NO: 1**; X-12 peptide is residues 344 - 356 of **SEQ ID NO: 1**; X-13 peptide is residues 348 - 360 of **SEQ ID NO: 1**; X-14 peptide is residues 352 - 364 of **SEQ ID NO: 1**; X-15 peptide is residues 344 - 357 **of SEQ ID NO: 1**; X-16 peptide is residues 348 - 361 of **SEQ ID NO: 1;** X-17 peptide is residues 352 - 365 of **SEQ ID NO: 1;** X-18 peptide is residues 344 - 358 of **SEQ ID NO: 1;** X-19 peptide is residues 348 - 362 of **SEQ ID NO: 1;** X-20 peptide is residues 352 - 366 of **SEQ ID NO: 1;** X-21 peptide is residues 344 - 359 of **SEQ ID NO: 1;** X-22 peptide is residues 348 - 363 of **SEQ ID NO: 1;** X-23 peptide is residues 344 - 360 of **SEQ ID NO: 1;** X-24 peptide is residues 348 - 364 of **SEQ ID NO: 1;** X-25 peptide is residues 344 - 361 of **SEQ ID NO: 1;** X-26 peptide is residues 348 - 365 of **SEQ ID NO: 1;** X-27 peptide is residues 344 - 362 of **SEQ ID NO: 1;** X-28 peptide is residues 348 - 366 of **SEQ ID NO: 1;** X-29 peptide is residues 344 - 363 of **SEQ ID NO: 1;** X-30 peptide is residues 344 - 364 of **SEQ ID NO: 1;** X-31 peptide is residues 344 - 365 of **SEQ ID NO: 1;** X-32 peptide is residues 344 - 366 of **SEQ ID NO: 1;** X-33 peptide is a scramble/random sequence; 'Y' = y-axis; y-axis peptide sequences depicted in columns Y1-Y19 are as follows: Y-1 peptide is residues 131 - 140 of **SEQ ID NO: 1;** Y-2 peptide is residues 135 - 144 of **SEQ ID NO: 1;** Y-3 peptide is residues 139 - 148 of **SEQ ID NO: 1;** Y-4 peptide is residues 131 - 141 of **SEQ ID NO: 1;** Y-5 peptide is residues 135 - 145 of **SEQ ID NO: 1;** Y-6 peptide is residues 139 - 149 of **SEQ ID NO: 1;** Y-7 peptide is residues 131 - 142 of **SEQ ID NO: 1;** Y-8 peptide is residues 135 - 146 of **SEQ ID NO: 1;** Y-9 peptide is residues 131 - 143 of **SEQ ID NO: 1;** Y-10 peptide is residues135 - 147 of **SEQ ID NO: 1;** Y-11 peptide is residues 131 - 144 of **SEQ ID NO: 1;** Y-12 peptide is residues 135 - 148 of **SEQ ID NO: 1;** Y-13 peptide is residues 131 - 145 of **SEQ ID NO: 1;** Y-14 peptide is residues 135 - 149 of **SEQ ID NO: 1;** Y-15 peptide is residues 131 - 146 of **SEQ ID NO: 1;** Y-16 peptide is residues 131 - 147 of **SEQ ID NO: 1;** Y-17 peptide is residues 131 - 148 of **SEQ ID NO: 1;** Y-18 peptide is residues 131 - 149 of **SEQ ID NO: 1;** Y-19 peptide is a scramble/random sequence.
**Figure 13** is a scatter plot matrix of all results obtained on the peptides of set 1;
**Figure 14** shows results of 2D electrophoresis and western blotting which demonstrate that MIL38 and anti-GPC-1 antibodies show overlapping reactivity on 2D gel western blot;
**Figure 15** shows western blots of immunoprecipitations conducted on DU-145 prostate cancer or C3 (MIL38 negative) cell membrane protein extracts with either MIL38 or anti-GPC-1 antibodies; and
**Figure 16** shows western blots of immunoprecipitations demonstrating that MIL38 antibody can detect glypican-1 in the plasma (**Figure 16A**) of prostate cancer patients and in extracts of prostate cancers (**Figure 16B**). **Figure 16A** lanes are: 046 IP NT- IP from prostate cancer plasma; 046 IP HepI- IP from prostate cancer plasma treated with heparinase; 042 IP NT- IP from normal control plasma; 042 IP HepI- IP from normal control plasma treated with heparinase; Magic Mark-commercial protein marker as molecular weight standard.

### Definitions

As used in this application, the singular form "a", "an" and "the" include plural references unless the context clearly dictates otherwise. For example, the phrase "an antibody" also includes multiple antibodies.
As used herein, the term "comprising" means "including." Variations of the word "comprising", such as "comprise" and "comprises," have correspondingly varied meanings. Thus, for example, a sample "comprising" antibody A may consist exclusively of antibody A or may include one or more additional components (e.g. antibody B).
As used herein the terms "multiple" and "plurality" mean more than one. In certain specific aspects or embodiments, multiple or plurality may mean 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or more, and any integer derivable therein, and any range derivable therein.
The term "epitope" as used herein refers to the specific portion(s) of an antigen which interact (e.g. bind) with one or more binding entities such as, for example, a protein, ligand, antibody, antibody fragment, or antibody derivative.
As used herein, the terms "antibody" and "antibodies" include IgG (including IgG1, IgG2, IgG3, and IgG4), IgA (including IgA1 and IgA2), IgD, IgE, IgM, and IgY, whole antibodies, including single-chain whole antibodies, and antigen-binding fragments thereof. Antigen-binding antibody fragments include, but are not limited to, Fv, Fab, Fab' and F(ab')2, Fd, single-chain Fvs (scFv), single-chain antibodies, disulfide-linked Fvs (sdFv) and fragments comprising either a VL or VH domain. The antibodies may be from any animal origin or appropriate production host. Antigen-binding antibody fragments, including single-chain antibodies, may comprise the variable region/s alone or in combination with the entire or partial of the following: hinge region, CH1, CH2, and CH3 domains. Also included are any combinations of variable region/s and hinge region, CH1, CH2, and CH3 domains. Antibodies may be monoclonal, polyclonal, chimeric, multispecific, humanised, and human monoclonal and polyclonal antibodies which specifically bind the biological molecule. The antibody may be a bi-specific antibody, avibody, diabody, tribody, tetrabody, nanobody, single domain antibody, VHH domain, human antibody, fully humanized antibody, partially humanized antibody, anticalin, adnectin, or affibody.
As used herein the term "monoclonal antibody" refers to an antibody that recognises a single antigenic epitope, and that is obtained from a population of substantially homogeneous antibodies which bind specifically to the same antigenic epitope, and are identical with the potential exception of naturally occurring mutation/s that may be present in minor amounts.
As used herein, the term "humanised antibody" refers to forms of antibodies that contain sequences from human antibodies as well as non-human antibodies (e.g. murine antibodies). For example, a humanised antibody can comprise substantially all of at least one and typically two variable domains, in which all/substantially all of the hypervariable loops correspond to those of a non-human immunoglobulin and all/substantially all of the FR regions are from the human immunoglobulin sequence. The humanised antibody may optionally also comprise at least a portion of an immunoglobulin constant region (Fc) which may typically be that of a human immunoglobulin.
As used herein, the term "chimeric antibody" refers to an antibody which exhibits a desired biological activity, and in which a portion of the light chain and/or heavy chain is identical to or homologous with corresponding sequences in antibodies derived from a given/specific species, while the remaining chain/s is/are identical to or homologous with corresponding sequences in antibodies derived from another different species. For example, a chimeric antibody may comprise variable regions that are derived from a first species and comprise constant regions that are derived from a second species. Chimeric antibodies can be constructed for example by genetic engineering from immunoglobulin gene segments belonging to different species.
As used herein, the term "hybridoma" refers to a cell produced by the fusion of an immortal cell (e.g. a multiple myeloma cell) and an antibody-producing cell (e.g. a B lymphocyte), which is capable of producing monoclonal antibodies of a single binding specificity.
As used herein, the terms "binding specifically" and "specifically binding" in reference to an antibody, antibody variant, antibody derivative, antigen binding fragment, and the like refers to its capacity to bind to a given target molecule preferentially over other non-target molecules. For example, if the antibody, antibody variant, antibody derivative, or antigen binding fragment ("molecule A") is capable of "binding specifically" or "specifically binding" to a given target molecule ("molecule B"), molecule A has the capacity to discriminate between molecule B and any other number of potential alternative binding partners. Accordingly, when exposed to a plurality of different but equally accessible molecules as potential binding partners, molecule A will selectively bind to molecule B and other alternative potential binding partners will remain substantially unbound by molecule A. In general, molecule A will preferentially bind to molecule B at least 10-fold, preferably 50-fold, more preferably 100-fold, and most preferably greater than 100-fold more frequently than other potential binding partners. Molecule A may be capable of binding to molecules that are not molecule B at a weak, yet detectable level. This is commonly known as background binding and is readily discernible from molecule B-specific binding, for example, by use of an appropriate control.
As used herein, the term "subject" includes any animal of economic, social or research importance including bovine, equine, ovine, primate, avian and rodent species. Hence, a "subject" may be a mammal such as, for example, a human or a non-human mammal.
As used herein, the term "isolated" in reference to a biological molecule (e.g. an antibody) is a biological molecule that is free from at least some of the components with which it naturally occurs.
As used herein, the terms "protein", "peptide" and "polypeptide" each refer to a polymer made up of amino acids linked together by peptide bonds and are used interchangeably. For the purposes of the present invention a "polypeptide" may constitute a full length protein or a portion of a full length protein, and there is no intended difference in the meaning of a "peptide" and a "polypeptide".
As used herein a "conservative" amino acid substitution refers to the replacement of a given amino acid residue in a sequence of amino acids with another, different amino acid residue of a similar size and/or of similar chemical properties. Non-limiting examples of conservative amino acid substitutions include: A (Ala) substituted with S (Ser); R (arg) substituted with K (lys); N (asn) substituted with Q (gln) or H (his); D (asp) substituted with E (glu); Q (gln) substituted with N (asn); C (cys) substituted with S (ser); E (glu) substituted with D (asp); G (gly) substituted with P (pro).
As used herein, the term "polynucleotide" refers to a single- or double-stranded polymer of deoxyribonucleotide bases, ribonucleotide bases, known analogues or natural nucleotides, or mixtures thereof.
As used herein the term "binding entity" encompasses any molecule capable of binding specifically to a GPC-1 epitope or mimic thereof as described herein. Non-limiting examples of binding entities include polypeptides such as, for example, antibodies.
As used herein, the term "kit" refers to any delivery system for delivering materials. Such delivery systems include systems that allow for the storage, transport, or delivery of reaction reagents (for example labels, reference samples, supporting material, etc. in the appropriate containers) and/or supporting materials (for example, buffers, written instructions for performing an assay etc.) from one location to another. For example, kits may include one or more enclosures, such as boxes, containing the relevant reaction reagents and/or supporting materials.
As used herein, the term "positive control element" in the context of a detection method or detection assay refers to an element that, when used in the method or assay, directly or indirectly provides a positive/affirmative signal, and thereby at least in part or wholly validates that the method or assay is capable of functioning correctly.
It will be understood that use of the term "between" herein when referring to a range of numerical values encompasses the numerical values at each endpoint of the range. For example, a polypeptide of between 10 residues and 20 residues in length is inclusive of a polypeptide of 10 residues in length and a polypeptide of 20 residues in length.

Any description of prior art documents herein, or statements herein derived from or based on those documents, is not an admission that the documents or derived statements are part of the common general knowledge of the relevant art.

### Detailed Description

The present inventors have identified specific epitopes within glypican-1 (GPC-1) advantageous for detecting and quantifying GPC-1 levels when using binding entities such as antibodies. Although useful for many purposes, these epitopes and combinations thereof can be targeted in diagnostic assays for prostate cancer seeking to quantify GPC-1 levels.

Accordingly, the present invention relates to GPC-1 epitopes and components thereof; combinations of said epitopes and/or epitope components; binding entities capable of specifically targeting the epitopes, components and/or combinations; compositions, kits and other entities comprising the epitopes, components and/or combinations; methods for generating binding entities capable of specifically targeting the epitopes, components and/or combinations; and diagnostic methods for prostate cancer requiring detection of the epitopes, components and/or combinations.

### Glypican-1 (GPC-1)

The present invention arises from a series of epitopes in glypican-1 heparan sulfate proteoglycan (GPC-1) which are targeted by specific binding entities (e.g. antibodies).

The epitopes may be present in a mammalian GPC-1 protein such as, for example, a bovine, equine, ovine, primate or rodent species. Hence, the epitopes may be present in a mammalian GPC-1 protein such as, for example, a human or a non-human mammal (e.g. a dog GPC-1 protein).

Additionally or alternatively, the epitopes may be present in a non-mammalian GPC-1 protein such as, for example, an avian GPC-1 protein.

The epitopes may be present in a human GPC-1 protein (e.g. as defined by a sequence set forth in any one of: NCBI reference sequence accession no. NP_002072.2, GenBank accession no. AAH51279.1, GenBank accession no. AAA98132.1, GenBank accession no. EAW71184.1, UniProtKB/Swiss-Prot accession no. P35052.2, and/or SEQ **ID** NO: 14).

It will be also understood that the epitopes may be present in a GPC-1 variant (e.g. a GPC-1 isoform, splice variant, or allotype). The epitopes may be present in cell-surface bound and/or secreted forms of GPC-1.

### Epitopes

### - Glypican-1 epitopes

Disclosed herein areGPC-1 epitopes and components thereof, and also combinations of said epitopes and/or epitope components.

In some embodiments, a GPC-1 epitope according to the present invention may comprise or consist of any one or more of the epitopes set out in **Table 1** below.

**Table 1: Non-limiting examples of GPC-1 epitopes according to the present invention**

| **SEQ ID NO** | **EPITOPE SEQUENCE** |
|---|---|
| **1** | KVNPQGPGPEEK (or a variant or fragment thereof) |
| **2** | VNPQGPGPEEK (or a variant or fragment thereof) |
| **3** | VNPQGPGPEE (or a variant or fragment thereof) |
| **4** | NPQGPGPEE (or a variant or fragment thereof) |
| **5** | KVNPQGPGPE (or a variant or fragment thereof) |
| **6** | KVNPQGPGP (or a variant or fragment thereof) |
| **7** | TQNARAFRD (or a variant or fragment thereof) |
| **8** | TQNARA (or a variant or fragment thereof) |
| **9** | ALSTASDDR (or a variant or fragment thereof) |
| **10** | PRERPP (or a variant or fragment thereof) |
| **11** | QDASDDGSGS (or a variant or fragment thereof) |
| **12** | CGELYTQNARAFRDLCGNPKVNPQGPGPEEKRRRGC (or a variant or fragment thereof) |
| **13** | LGPECSRAVMK (or a variant or fragment thereof) |

In certain embodiments an epitope of the present invention comprises or consists of a plurality of segments. These epitopes may be linear or discontinuous. Non-limiting examples of epitopes comprising a plurality of segments are set out in **Table 2** below.

**Table 2: Further non-limiting examples of GPC-1 epitopes according to the present invention**

| **SEQ ID NOs** | **EPITOPE SEGMENT SEQUENCES** |
|---|---|
| **1** | 1st segment: KVNPQGPGPEEK (or a variant or fragment thereof) |
| **7** | 2nd segment: TQNARAFRD (or a variant or fragment thereof) |
| **6** | 1 st segment: KVNPQGPGP (or a variant or fragment thereof) |
| **7** | 2nd segment: TQNARAFRD (or a variant or fragment thereof) |
| **4** | 1 st segment: NPQGPGPEE (or a variant or fragment thereof) |
| **7** | 2nd segment: TQNARAFRD (or a variant or fragment thereof) |
| **3** | 1st segment: VNPQGPGPEE (or a variant or fragment thereof) |
| **7** | 2nd segment: TQNARAFRD (or a variant or fragment thereof) |
| **2** | 1 st segment: VNPQGPGPEEK (or a variant or fragment thereof) |
| **7** | 2nd segment: TQNARAFRD (or a variant or fragment thereof) |
| **5** | 1 st segment: KVNPQGPGPE (or a variant or fragment thereof) |
| **7** | 2nd segment: TQNARAFRD (or a variant or fragment thereof) |
| | |
| **1** | 1st segment: KVNPQGPGPEEK (or a variant or fragment thereof) |
| **8** | 2nd segment: TQNARA (or a variant or fragment thereof) |
| **6** | 1st segment: KVNPQGPGP (or a variant or fragment thereof) |
| **8** | 2nd segment: TQNARA (or a variant or fragment thereof) |
| **4** | 1st segment: NPQGPGPEE (or a variant or fragment thereof) |
| **8** | 2nd segment: TQNARA (or a variant or fragment thereof) |
| **3** | 1st segment: VNPQGPGPEE (or a variant or fragment thereof) |
| **8** | 2nd segment: TQNARA (or a variant or fragment thereof) |
| **2** | 1st segment: VNPQGPGPEEK (or a variant or fragment thereof) |
| **8** | 2nd segment: TQNARA (or a variant or fragment thereof) |
| **5** | 1st segment: KVNPQGPGPE (or a variant or fragment thereof) |
| **8** | 2nd segment: TQNARA (or a variant or fragment thereof) |
| | |
| **1** | 1st segment: KVNPQGPGPEEK (or a variant or fragment thereof) |
| **9** | 2nd segment: ALSTASDDR (or a variant or fragment thereof) |
| **6** | 1st segment: KVNPQGPGP (or a variant or fragment thereof) |
| **9** | 2nd segment: ALSTASDDR (or a variant or fragment thereof) |
| **4** | 1st segment: NPQGPGPEE (or a variant or fragment thereof) |
| **9** | 2nd segment: ALSTASDDR (or a variant or fragment thereof) |
| **3** | 1st segment: VNPQGPGPEE (or a variant or fragment thereof) |
| **9** | 2nd segment: ALSTASDDR (or a variant or fragment thereof) |
| **2** | 1 st segment: VNPQGPGPEEK (or a variant or fragment thereof) |
| **9** | 2nd segment: ALSTASDDR (or a variant or fragment thereof) |
| **5** | 1st segment: KVNPQGPGPE (or a variant or fragment thereof) |
| **9** | 2nd segment: ALSTASDDR (or a variant or fragment thereof) |
| | |
| **1** | 1st segment: KVNPQGPGPEEK (or a variant or fragment thereof) |
| **11** | 2nd segment: QDASDDGSGS (or a variant or fragment thereof) |
| **6** | 1st segment: KVNPQGPGP (or a variant or fragment thereof) |
| **11** | 2nd segment: QDASDDGSGS (or a variant or fragment thereof) |
| **4** | 1st segment: NPQGPGPEE (or a variant or fragment thereof) |
| **11** | 2nd segment: QDASDDGSGS (or a variant or fragment thereof) |
| **3** | 1st segment: VNPQGPGPEE (or a variant or fragment thereof) |
| **11** | 2nd segment: QDASDDGSGS (or a variant or fragment thereof) |
| **2** | 1st segment: VNPQGPGPEEK (or a variant or fragment thereof) |
| **11** | 2nd segment: QDASDDGSGS (or a variant or fragment thereof) |
| **5** | 1st segment: KVNPQGPGPE (or a variant or fragment thereof) |
| **11** | 2nd segment: QDASDDGSGS (or a variant or fragment thereof) |
| | |
| **1** | 1st segment: KVNPQGPGPEEK (or a variant or fragment thereof) |
| **13** | 2nd segment: LGPECSRAVMK (or a variant or fragment thereof) |
| **6** | 1st segment: KVNPQGPGP (or a variant or fragment thereof) |
| **13** | 2nd segment: LGPECSRAVMK (or a variant or fragment thereof) |
| **4** | 1st segment: NPQGPGPEE (or a variant or fragment thereof) |
| **13** | 2nd segment: LGPECSRAVMK (or a variant or fragment thereof) |
| **3** | 1st segment: VNPQGPGPEE (or a variant or fragment thereof) |
| **13** | 2nd segment: LGPECSRAVMK (or a variant or fragment thereof) |
| **2** | 1st segment: VNPQGPGPEEK (or a variant or fragment thereof) |
| **13** | 2nd segment: LGPECSRAVMK (or a variant or fragment thereof) |
| **5** | 1st segment: KVNPQGPGPE (or a variant or fragment thereof) |
| **13** | 2nd segment: LGPECSRAVMK (or a variant or fragment thereof) |
| | |
| **1** | 1st segment: KVNPQGPGPEEK (or a variant or fragment thereof) |
| **10** | 2nd segment: PRERPP (or a variant or fragment thereof) |
| **6** | 1st segment: KVNPQGPGP (or a variant or fragment thereof) |
| **10** | 2nd segment: PRERPP (or a variant or fragment thereof) |
| **4** | 1st segment: NPQGPGPEE (or a variant or fragment thereof) |
| **10** | 2nd segment: PRERPP (or a variant or fragment thereof) |
| **3** | 1st segment: VNPQGPGPEE (or a variant or fragment thereof) |
| **10** | 2nd segment: PRERPP (or a variant or fragment thereof) |
| **2** | 1st segment: VNPQGPGPEEK (or a variant or fragment thereof) |
| **10** | 2nd segment: PRERPP (or a variant or fragment thereof) |
| **5** | 1st segment: KVNPQGPGPE (or a variant or fragment thereof) |
| **10** | 2nd segment: PRERPP (or a variant or fragment thereof) |
| | |
| **11** | 1st segment: QDASDDGSGS (or a variant or fragment thereof) |
| **13** | 2nd segment: LGPECSRAVMK (or a variant or fragment thereof) |
| **10** | 1st segment: PRERPP (or a variant or fragment thereof) |
| **13** | 2nd segment: LGPECSRAVMK (or a variant or fragment thereof) |
| **9** | 1st segment: ALSTASDDR (or a variant or fragment thereof) |
| **13** | 2nd segment: LGPECSRAVMK (or a variant or fragment thereof) |
| **8** | 1st segment: TQNARA (or a variant or fragment thereof) |
| **13** | 2nd segment: LGPECSRAVMK (or a variant or fragment thereof) |
| **7** | 1st segment: TQNARAFRD (or a variant or fragment thereof) |
| **13** | 2nd segment: LGPECSRAVMK (or a variant or fragment thereof) |
| | |
| **7** | 1st segment: TQNARAFRD (or a variant or fragment thereof) |
| **9** | 2nd segment: ALSTASDDR (or a variant or fragment thereof) |
| **8** | 1st segment: TQNARA (or a variant or fragment thereof) |
| **9** | 2nd segment: ALSTASDDR (or a variant or fragment thereof) |
| **10** | 1 st segment: PRERPP (or a variant or fragment thereof) |
| **9** | 2nd segment: ALSTASDDR (or a variant or fragment thereof) |
| **11** | 1st segment: QDASDDGSGS (or a variant or fragment thereof) |
| **9** | 2nd segment: ALSTASDDR (or a variant or fragment thereof) |
| | |
| **7** | 1st segment: TQNARAFRD (or a variant or fragment thereof) |
| **10** | 2nd segment: PRERPP (or a variant or fragment thereof) |
| **8** | 1st segment: TQNARA (or a variant or fragment thereof) |
| **10** | 2nd segment: PRERPP (or a variant or fragment thereof) |
| **11** | 1st segment: QDASDDGSGS (or a variant or fragment thereof) |
| **10** | 2nd segment: PRERPP (or a variant or fragment thereof) |
| | |
| **7** | 1st segment: TQNARAFRD (or a variant or fragment thereof) |
| **11** | 2nd segment: QDASDDGSGS (or a variant or fragment thereof) |
| **8** | 1st segment: TQNARA (or a variant or fragment thereof) |
| **11** | 2nd segment: QDASDDGSGS (or a variant or fragment thereof) |
| | |
| **1** | 1st segment: KVNPQGPGPEEK (or a variant or fragment thereof) |
| **10** | 2nd segment: PRERPP (or a variant or fragment thereof) |
| **11** | 3rd segment: QDASDDGSGS (or a variant or fragment thereof) |
| **6** | 1st segment: KVNPQGPGP (or a variant or fragment thereof) |
| **10** | 2nd segment: PRERPP (or a variant or fragment thereof) |
| **11** | 3rd segment: QDASDDGSGS (or a variant or fragment thereof) |
| **4** | 1st segment: NPQGPGPEE (or a variant or fragment thereof) |
| **10** | 2nd segment: PRERPP (or a variant or fragment thereof) |
| **11** | 3rd segment: QDASDDGSGS (or a variant or fragment thereof) |
| **3** | 1st segment: VNPQGPGPEE (or a variant or fragment thereof) |
| **10** | 2nd segment: PRERPP (or a variant or fragment thereof) |
| **11** | 3rd segment: QDASDDGSGS (or a variant or fragment thereof) |
| **2** | 1st segment: VNPQGPGPEEK (or a variant or fragment thereof) |
| **10** | 2nd segment: PRERPP (or a variant or fragment thereof) |
| **11** | 3rd segment: QDASDDGSGS (or a variant or fragment thereof) |
| **5** | 1st segment: KVNPQGPGPE (or a variant or fragment thereof) |
| **10** | 2nd segment: PRERPP (or a variant or fragment thereof) |
| **11** | 3rd segment: QDASDDGSGS (or a variant or fragment thereof) |
| **1** | 1st segment: KVNPQGPGPEEK (or a variant or fragment thereof) |
| **10** | 2nd segment: PRERPP (or a variant or fragment thereof) |
| **11** | 3rd segment: QDASDDGSGS (or a variant or fragment thereof) |

In other embodiments, there is disclosed combinations of distinct epitopes that comprise or consist of a plurality of discrete epitopes. These discrete epitopes may be linear or discontinuous. Non-limiting examples of epitope combinations comprising a plurality of distinct epitopes are set out in **Table 3** below.

**Table 3: Non-limiting examples of GPC-1 epitope combinations according to the present invention**

| **SEQ ID NOs** | **EPITOPE COMBINATION SEQUENCES** |
|---|---|
| **1** | 1st epitope: KVNPQGPGPEEK (or a variant or fragment thereof) |
| **7** | 2nd epitope: TQNARAFRD (or a variant or fragment thereof) |
| **6** | 1st epitope: KVNPQGPGP (or a variant or fragment thereof) |
| **7** | 2nd epitope: TQNARAFRD (or a variant or fragment thereof) |
| **4** | 1st epitope: NPQGPGPEE (or a variant or fragment thereof) |
| **7** | 2nd epitope: TQNARAFRD (or a variant or fragment thereof) |
| **3** | 1st epitope: VNPQGPGPEE (or a variant or fragment thereof) |
| **7** | 2nd epitope: TQNARAFRD (or a variant or fragment thereof) |
| **2** | 1st epitope: VNPQGPGPEEK (or a variant or fragment thereof) |
| **7** | 2nd epitope: TQNARAFRD (or a variant or fragment thereof) |
| **5** | 1st epitope: KVNPQGPGPE (or a variant or fragment thereof) |
| **7** | 2nd epitope: TQNARAFRD (or a variant or fragment thereof) |
| | |
| **1** | 1st epitope: KVNPQGPGPEEK (or a variant or fragment thereof) |
| **8** | 2nd epitope: TQNARA (or a variant or fragment thereof) |
| **6** | 1st epitope: KVNPQGPGP (or a variant or fragment thereof) |
| **8** | 2nd epitope: TQNARA (or a variant or fragment thereof) |
| **4** | 1st epitope: NPQGPGPEE (or a variant or fragment thereof) |
| **8** | 2nd epitope: TQNARA (or a variant or fragment thereof) |
| **3** | 1st epitope: VNPQGPGPEE (or a variant or fragment thereof) |
| **8** | 2nd epitope: TQNARA (or a variant or fragment thereof) |
| **2** | 1st epitope: VNPQGPGPEEK (or a variant or fragment thereof) |
| **8** | 2nd epitope: TQNARA (or a variant or fragment thereof) |
| **5** | 1st epitope: KVNPQGPGPE (or a variant or fragment thereof) |
| **8** | 2nd epitope: TQNARA (or a variant or fragment thereof) |
| | |
| **1** | 1st epitope: KVNPQGPGPEEK (or a variant or fragment thereof) |
| **9** | 2nd epitope: ALSTASDDR (or a variant or fragment thereof) |
| **6** | 1st epitope: KVNPQGPGP (or a variant or fragment thereof) |
| **9** | 2nd epitope: ALSTASDDR (or a variant or fragment thereof) |
| **4** | 1st epitope: NPQGPGPEE (or a variant or fragment thereof) |
| **9** | 2nd epitope: ALSTASDDR (or a variant or fragment thereof) |
| **3** | 1st epitope: VNPQGPGPEE (or a variant or fragment thereof) |
| **9** | 2nd epitope: ALSTASDDR (or a variant or fragment thereof) |
| **2** | 1st epitope: VNPQGPGPEEK (or a variant or fragment thereof) |
| **9** | 2nd epitope: ALSTASDDR (or a variant or fragment thereof) |
| **5** | 1st epitope: KVNPQGPGPE (or a variant or fragment thereof) |
| **9** | 2nd epitope: ALSTASDDR (or a variant or fragment thereof) |
| | |
| **1** | 1st epitope: KVNPQGPGPEEK (or a variant or fragment thereof) |
| **11** | 2nd epitope: QDASDDGSGS (or a variant or fragment thereof) |
| **6** | 1st epitope: KVNPQGPGP (or a variant or fragment thereof) |
| **11** | 2nd epitope: QDASDDGSGS (or a variant or fragment thereof) |
| **4** | 1st epitope: NPQGPGPEE (or a variant or fragment thereof) |
| **11** | 2nd epitope: QDASDDGSGS (or a variant or fragment thereof) |
| **3** | 1st epitope: VNPQGPGPEE (or a variant or fragment thereof) |
| **11** | 2nd epitope: QDASDDGSGS (or a variant or fragment thereof) |
| **2** | 1st epitope: VNPQGPGPEEK (or a variant or fragment thereof) |
| **11** | 2nd epitope: QDASDDGSGS (or a variant or fragment thereof) |
| **5** | 1st epitope: KVNPQGPGPE (or a variant or fragment thereof) |
| **11** | 2nd epitope: QDASDDGSGS (or a variant or fragment thereof) |
| | |
| **1** | 1st epitope: KVNPQGPGPEEK (or a variant or fragment thereof) |
| **13** | 2nd epitope: LGPECSRAVMK (or a variant or fragment thereof) |
| **6** | 1st epitope: KVNPQGPGP (or a variant or fragment thereof) |
| **13** | 2nd epitope: LGPECSRAVMK (or a variant or fragment thereof) |
| **4** | 1st epitope: NPQGPGPEE (or a variant or fragment thereof) |
| **13** | 2nd epitope: LGPECSRAVMK (or a variant or fragment thereof) |
| **3** | 1st epitope: VNPQGPGPEE (or a variant or fragment thereof) |
| **13** | 2nd epitope: LGPECSRAVMK (or a variant or fragment thereof) |
| **2** | 1st epitope: VNPQGPGPEEK (or a variant or fragment thereof) |
| **13** | 2nd epitope: LGPECSRAVMK (or a variant or fragment thereof) |
| **5** | 1st epitope: KVNPQGPGPE (or a variant or fragment thereof) |
| **13** | 2nd epitope: LGPECSRAVMK (or a variant or fragment thereof) |
| | |
| **1** | 1st epitope: KVNPQGPGPEEK (or a variant or fragment thereof) |
| **10** | 2nd epitope: PRERPP (or a variant or fragment thereof) |
| **6** | 1st epitope: KVNPQGPGP (or a variant or fragment thereof) |
| **10** | 2nd epitope: PRERPP (or a variant or fragment thereof) |
| **4** | 1st epitope: NPQGPGPEE (or a variant or fragment thereof) |
| **10** | 2nd epitope: PRERPP (or a variant or fragment thereof) |
| **3** | 1st epitope: VNPQGPGPEE (or a variant or fragment thereof) |
| **10** | 2nd epitope: PRERPP (or a variant or fragment thereof) |
| **2** | 1st epitope: VNPQGPGPEEK (or a variant or fragment thereof) |
| **10** | 2nd epitope: PRERPP (or a variant or fragment thereof) |
| **5** | 1st epitope: KVNPQGPGPE (or a variant or fragment thereof) |
| **10** | 2nd epitope: PRERPP (or a variant or fragment thereof) |
| | |
| **11** | 1st epitope: QDASDDGSGS (or a variant or fragment thereof) |
| **13** | 2nd epitope: LGPECSRAVMK (or a variant or fragment thereof) |
| **10** | 1st epitope: PRERPP (or a variant or fragment thereof) |
| **13** | 2nd epitope: LGPECSRAVMK (or a variant or fragment thereof) |
| **9** | 1st epitope: ALSTASDDR (or a variant or fragment thereof) |
| **13** | 2nd epitope: LGPECSRAVMK (or a variant or fragment thereof) |
| **8** | 1st epitope: TQNARA (or a variant or fragment thereof) |
| **13** | 2nd epitope: LGPECSRAVMK (or a variant or fragment thereof) |
| **7** | 1st epitope: TQNARAFRD (or a variant or fragment thereof) |
| **13** | 2nd epitope: LGPECSRAVMK (or a variant or fragment thereof) |
| | |
| **7** | 1st epitope: TQNARAFRD (or a variant or fragment thereof) |
| **9** | 2nd epitope: ALSTASDDR (or a variant or fragment thereof) |
| **8** | 1st epitope: TQNARA (or a variant or fragment thereof) |
| **9** | 2nd epitope: ALSTASDDR (or a variant or fragment thereof) |
| **10** | 1st epitope: PRERPP (or a variant or fragment thereof) |
| **9** | 2nd epitope: ALSTASDDR (or a variant or fragment thereof) |
| **11** | 1st epitope: QDASDDGSGS (or a variant or fragment thereof) |
| **9** | 2nd epitope: ALSTASDDR (or a variant or fragment thereof) |
| | |
| **7** | 1st epitope: TQNARAFRD (or a variant or fragment thereof) |
| **10** | 2nd epitope: PRERPP (or a variant or fragment thereof) |
| **8** | 1st epitope: TQNARA (or a variant or fragment thereof) |
| **10** | 2nd epitope: PRERPP (or a variant or fragment thereof) |
| **11** | 1st epitope: QDASDDGSGS (or a variant or fragment thereof) |
| **10** | 2nd epitope: PRERPP (or a variant or fragment thereof) |
| | |
| **7** | 1st epitope: TQNARAFRD (or a variant or fragment thereof) |
| **11** | 2nd epitope: QDASDDGSGS (or a variant or fragment thereof) |
| **8** | 1st epitope: TQNARA (or a variant or fragment thereof) |
| **11** | 2nd epitope: QDASDDGSGS (or a variant or fragment thereof) |
| | |
| **1** | 1st epitope: KVNPQGPGPEEK (or a variant or fragment thereof) |
| **10** | 2nd epitope: PRERPP (or a variant or fragment thereof) |
| **11** | 3rd epitope: QDASDDGSGS (or a variant or fragment thereof) |
| **6** | 1st epitope: KVNPQGPGP (or a variant or fragment thereof) |
| **10** | 2nd epitope: PRERPP (or a variant or fragment thereof) |
| **11** | 3rd epitope: QDASDDGSGS (or a variant or fragment thereof) |
| **4** | 1st epitope: NPQGPGPEE (or a variant or fragment thereof) |
| **10** | 2nd epitope: PRERPP (or a variant or fragment thereof) |
| **11** | 3rd epitope: QDASDDGSGS (or a variant or fragment thereof) |
| **3** | 1st epitope: VNPQGPGPEE (or a variant or fragment thereof) |
| **10** | 2nd epitope: PRERPP (or a variant or fragment thereof) |
| **11** | 3rd epitope: QDASDDGSGS (or a variant or fragment thereof) |
| **2** | 1st epitope: VNPQGPGPEEK (or a variant or fragment thereof) |
| **10** | 2nd epitope: PRERPP (or a variant or fragment thereof) |
| **11** | 3rd epitope: QDASDDGSGS (or a variant or fragment thereof) |
| **5** | 1st epitope: KVNPQGPGPE (or a variant or fragment thereof) |
| **10** | 2nd epitope: PRERPP (or a variant or fragment thereof) |
| **11** | 3rd epitope: QDASDDGSGS (or a variant or fragment thereof) |
| **1** | 1st epitope: KVNPQGPGPEEK (or a variant or fragment thereof) |
| **10** | 2nd epitope: PRERPP (or a variant or fragment thereof) |
| **11** | 3rd epitope: QDASDDGSGS (or a variant or fragment thereof) |

A combination of epitopes may comprise prostate-specific antigen (PSA), also known as gamma-seminoprotein or kallikrein-3 (KLK3). Accordingly, a combination of epitopes may comprise any one of the epitopes listed in **Table 1** or **Table 2** in combination with PSA, or any one of the epitope combinations listed in **Table 3** further combined with PSA.

### - Variants

Provided herein are variants of the GPC-1 epitopes.

The variants may comprise conservative or non-conservative amino acid substitution(s), as known to those of ordinary skill in the art.

In some embodiments, a variant of an epitope of the present invention may have a specified percentage of amino acid residues that are the same (percentage of "sequence identity"), over a specified region, or, when not specified, over the entire sequence. Accordingly, a "variant" of a GPC-1 epitope disclosed herein may share at least 40%, 45%, 50%, 55%, 60%, 65%, 70%, 75%, 80%, 83%, 85%, 88%, 90%, 93%, 95%, 96%, 97%, 98% or 99% sequence identity with the sequence of a GPC-1 epitope described herein.

The variant may retain identical, substantially identical, or altered biological activity in comparison to the GPC-1 epitope sequence from which the variant arises.

The variant may be a homologue GPC-1 epitope from a different family, genus or species having identical or substantially identical biological function or activity to the GPC-1 epitope sequence from which the variant arises (e.g. those derived from other species of mammals).

Differences in sequence identity may arise from amino acid substitutions (e.g. conservative and/or non-conservative substitutions), insertions and/or deletions. A conservative amino acid substitution refers to a substitution or replacement of one amino acid for another amino acid with similar properties within a polypeptide chain, as well known to those of ordinary skill in the art. For example, the substitution of the charged amino acid glutamic acid (Glu) for the similarly charged amino acid aspartic acid (Asp) would be a conservative amino acid substitution.

The percentage of sequence identity between two sequences may be determined without difficulty using methods known to those of ordinary skill in the art. For example, the percentage of sequence identity between two sequences may be determined by comparing two optimally aligned sequences over a comparison window. The portion of the sequence in the comparison window may, for example, comprise deletions or additions (i.e. gaps) in comparison to the reference sequence (for example, a GPC-1 epitope sequence as described herein), which does not comprise deletions or additions, in order to align the two sequences optimally. A percentage of sequence identity may then be calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The level of sequence identity may be measured using sequence analysis software (e.g., Sequence Analysis Software Package, Genetics Computer Group, University of Wisconsin Biotechnology Center, 1710 University Ave., Madison, Wis. 53705). This software matches similar sequences by assigning degrees of homology to various substitutions, deletions, and other modifications. Other suitable examples computer software for measuring the degree of sequence identity between two or more sequences include, but are not limited to, CLUSTAL in the PC/Gene program (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA).

In relation to the above embodiments, including those set out in **Tables 1-3** above, **Table 4** below provides suitable and non-limiting examples of the variants referred to.

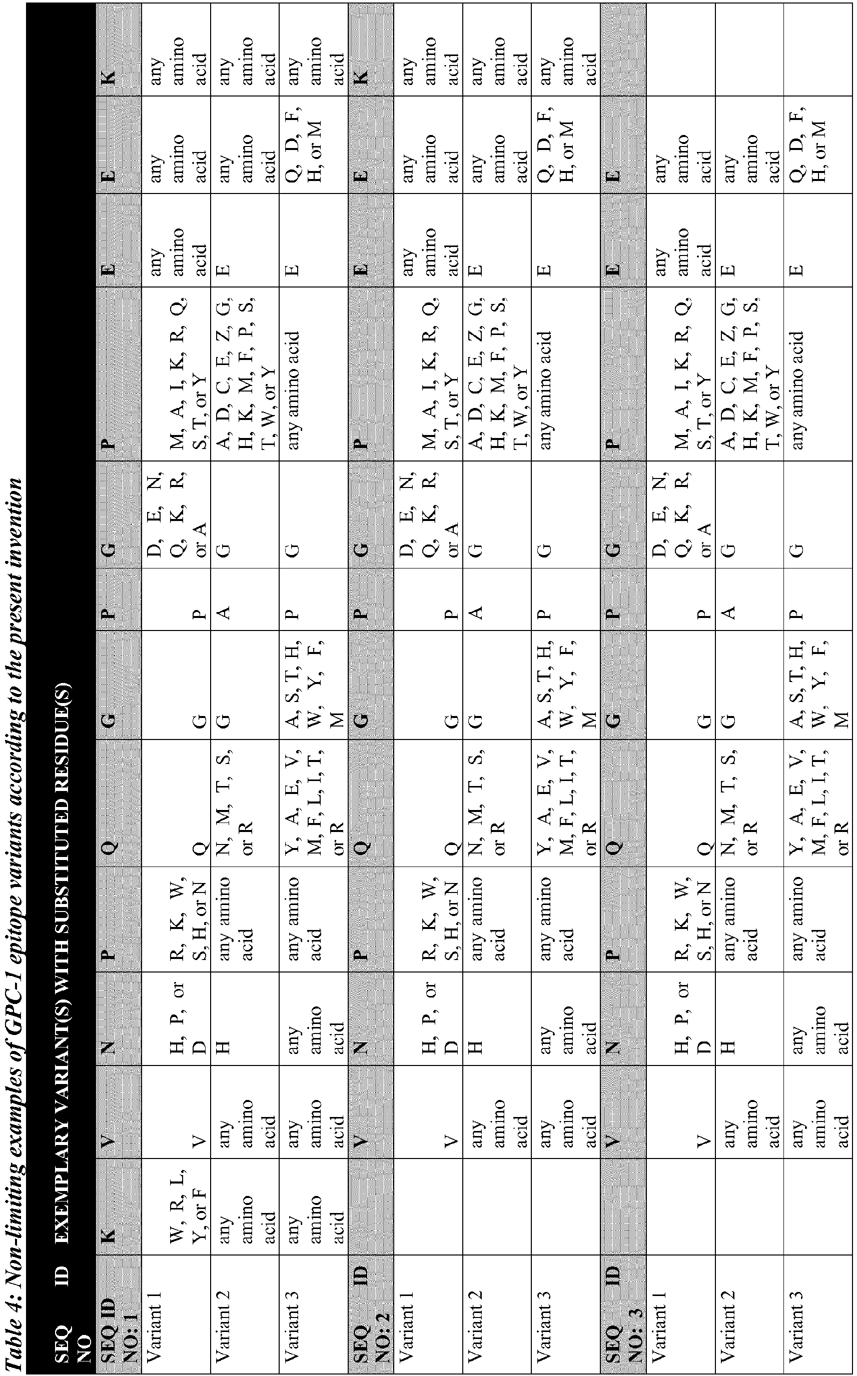

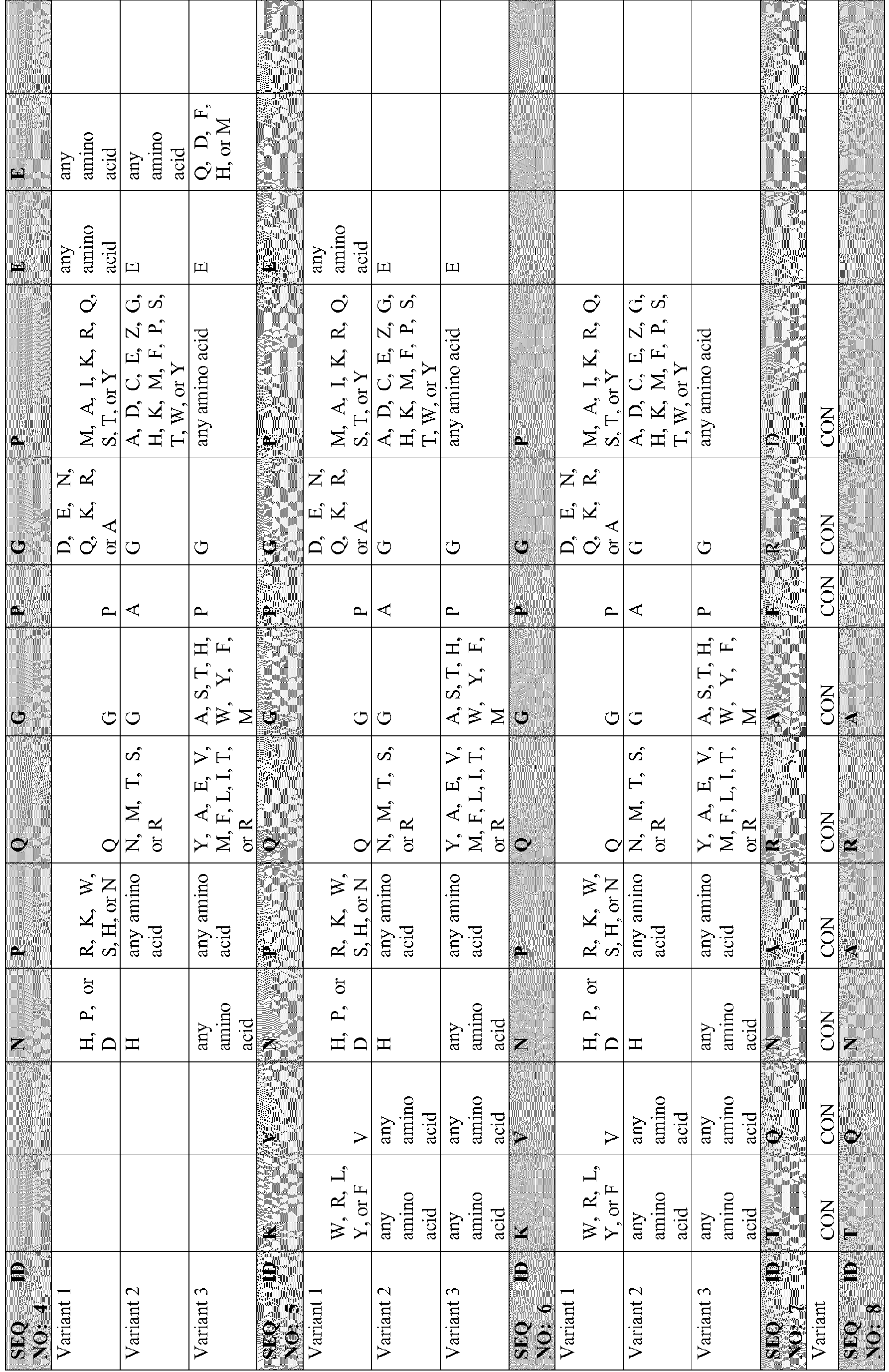

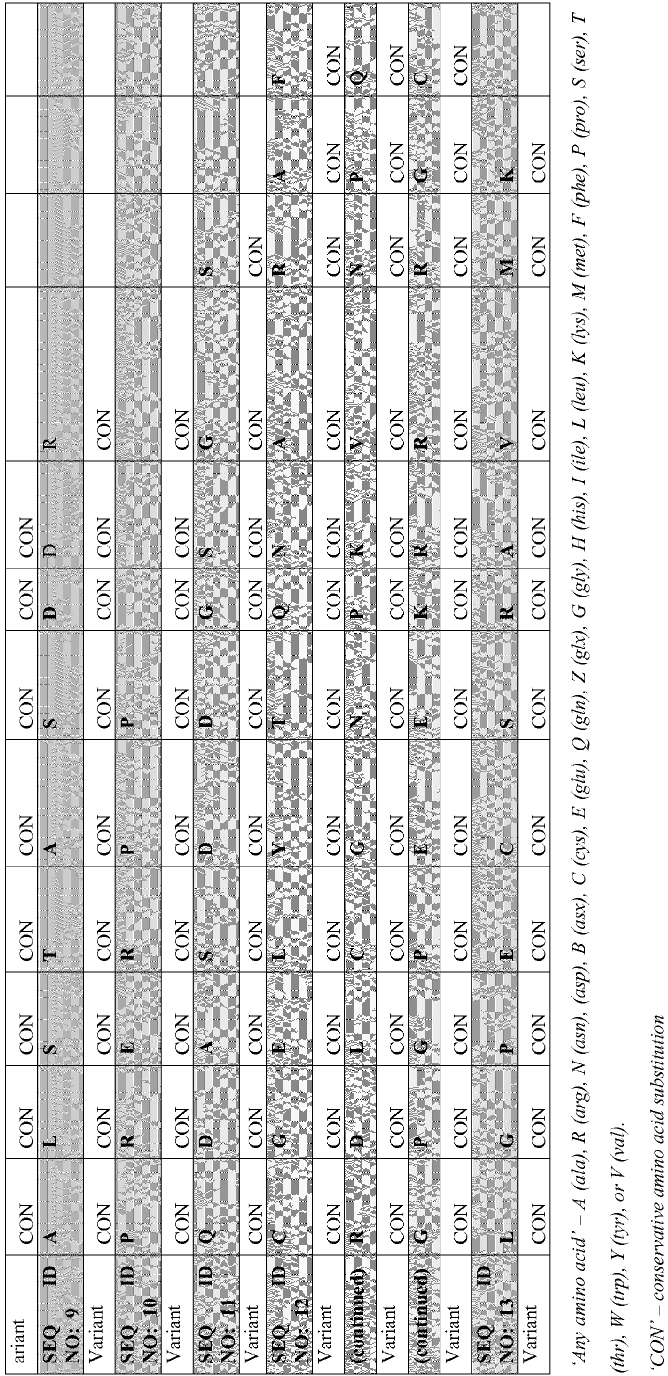

Accordingly, a variant of K**V**NP**QGP**GPEEK (**SEQ ID NO: 1**) may comprise V (val) at position 2, Q (gln) at position 5, G (gly) at position 6, and P (pro) at position 7. The variant may further comprise K (lys) at position 1, and/or N (asn) at position 3, and/or P (pro) at position 4. Additionally or alternatively, the variant may further comprise G (gly) at position 8, and/or P (pro) at position 9, and/or E (glu) at position 10. Additionally or alternatively, the variant may comprise one, two, three, four, five, six, seven or eight substituted residues. The substituted amino acid residue(s) may be at any one or more of positions 1, 3, 4, 8, 9, 10, 11, and/or 12 of **SEQ ID NO: 1.**

Alternatively, a variant of KVNPQ**G**P**G**P**E**EK (**SEQ ID NO: 1**) may comprise G (gly) at position 6, G (gly) at position 8, and E (glu) at position 10. The variant may further comprise N (asn) at position 3, and/or Q (gln) at position 5, and/or P (pro) at position 7. Additionally or alternatively, the variant may comprise one, two, three, four, five, six, seven, eight or nine substituted residues. The substituted amino acid residue(s) may be at any one or more of positions 1, 2, 3, 4, 5, 7, 9, 11, and/or 12 of **SEQ ID NO: 1.**

Alternatively, a variant of KVNPQ**GP**GP**E**EK (**SEQ ID NO: 1**) may comprise P (pro) at position 7, G (gly) at position 8, and E (glu) at position 10. The variant may further comprise Q (gln) at position 5, and/or G (gly) at position 6, and/or E (glu) at position 11. Additionally or alternatively, the variant may comprise one, two, three, four, five, six, seven, eight or nine substituted residues. The substituted amino acid residue(s) may be at any one or more of positions 1, 2, 3, 4, 5, 6, 9, 11, and/or 12 of **SEQ ID NO: 1.**

A variant of **V**NP**QGP**GPEEK (**SEQ ID NO: 2**) may comprise V (val) at position 1, Q (gln) at position 4, G (gly) at position 5, and P (pro) at position 6. The variant may further comprise N (asn) at position 2, and/or P (pro) at position 3. Additionally or alternatively, the variant may further comprise G (gly) at position 7, and/or P (pro) at position 8, and/or E (glu) at position 9. Additionally or alternatively, the variant may comprise one, two, three, four, five, six, or seven substituted residues. The substituted amino acid residue(s) may be at any one or more of positions 2, 3, 7, 8, 9, 10, and/or 11 of **SEQ ID NO: 2.**

Alternatively, a variant of VNPQ**G**P**G**P**E**EK (**SEQ ID NO: 2**) may comprise G (gly) at position 5, G (gly) at position 7, and E (glu) at position 9. The variant may further comprise N (asn) at position 2, and/or Q (gln) at position 4, and/or P (pro) at position 6. Additionally or alternatively, the variant may comprise one, two, three, four, five, six, seven, or eight substituted residues. The substituted amino acid residue(s) may be at any one or more of positions 1, 2, 3, 4, 6, 8, 10, and/or 11 of **SEQ ID NO: 2.**

Alternatively, a variant of VNPQG**PG**P**E**EK (**SEQ ID NO: 2**) may comprise P (pro) at position 6, G (gly) at position 7, and E (glu) at position 9. The variant may further comprise Q (gln) at position 4, and/or G (gly) at position 5, and/or E (glu) at position 10. Additionally or alternatively, the variant may comprise one, two, three, four, five, six, seven, or eight substituted residues. The substituted amino acid residue(s) may be at any one or more of positions 1, 2, 3, 4, 5, 8, 10, and/or 11of **SEQ ID NO: 2.**

A variant of **V**NP**QGP**GPEE (**SEQ ID NO: 3**) may comprise V (val) at position 1, Q (gln) at position 4, G (gly) at position 5, and P (pro) at position 6. The variant may further comprise N (asn) at position 2, and/or P (pro) at position 3. Additionally or alternatively, the variant may further comprise G (gly) at position 7, and/or P (pro) at position 8, and/or E (glu) at position 9. Additionally or alternatively, the variant may comprise one, two, three, four, five or six substituted residues. The substituted amino acid residue(s) may be at any one or more of positions 2, 3, 7, 8, 9, and/or 10 of **SEQ ID NO: 3.**

Alternatively, a variant of VNPQ**G**P**G**P**E**E (**SEQ ID NO: 3**) may comprise G (gly) at position 5, G (gly) at position 7, and E (glu) at position 9. The variant may further comprise N (asn) at position 2, and/or Q (gln) at position 4, and/or P (pro) at position 6. Additionally or alternatively, the variant may comprise one, two, three, four, five, six, or seven substituted residues. The substituted amino acid residue(s) may be at any one or more of positions 1, 2, 3, 4, 6, 8, and/or 10 of **SEQ ID NO: 3.**

Alternatively, a variant of VNPQG**PG**P**E**E (**SEQ ID NO: 3**) may comprise P (pro) at position 6, G (gly) at position 7, and E (glu) at position 9. The variant may further comprise Q (gln) at position 4, and/or G (gly) at position 5, and/or E (glu) at position 10. Additionally or alternatively, the variant may comprise one, two, three, four, five, six, or seven substituted residues. The substituted amino acid residue(s) may be at any one or more of positions 1, 2, 3, 4, 5, 8, and/or 10 of **SEQ ID NO: 3**.

A variant of NP**QGP**GPEE (**SEQ ID NO: 4**) may comprise Q (gln) at position 3, G (gly) at position 4, and P (pro) at position 5. The variant may further comprise N (asn) at position 1, and/or P (pro) at position 2. Additionally or alternatively, the variant may further comprise G (gly) at position 6, and/or P (pro) at position 7, and/or E (glu) at position 8. Additionally or alternatively, the variant may comprise one, two, three, four, five, or six substituted residues. The substituted amino acid residue(s) may be at any one or more of positions 1, 2, 6, 7, 8, and/or 9 of **SEQ ID NO: 4**.

Alternatively, a variant of NPQ**G**P**G**P**E**E (**SEQ ID NO: 4**) may comprise G (gly) at position 4, G (gly) at position 6, and E (glu) at position 8. The variant may further comprise N (asn) at position 1, and/or Q (gln) at position 3, and/or P (pro) at position 5. Additionally or alternatively, the variant may comprise one, two, three, four, five, or six substituted residues. The substituted amino acid residue(s) may be at any one or more of positions 1, 2, 3, 5, 7, and/or 9 of **SEQ ID NO: 4.**

Alternatively, a variant of NPQG**PG**P**E**E (**SEQ ID NO: 4**) may comprise P (pro) at position 5, G (gly) at position 6, and E (glu) at position 8. The variant may further comprise Q (gln) at position 3, and/or G (gly) at position 4, and/or E (glu) at position 9. Additionally or alternatively, the variant may comprise one, two, three, four, five, or six, substituted residues. The substituted amino acid residue(s) may be at any one or more of positions 1, 2, 3, 4, 7, and/or 9 of **SEQ ID NO: 4.**

A variant of K**V**NP**QGP**GPE (**SEQ ID NO: 5**) may comprise V (val) at position 2, Q (gln) at position 5, G (gly) at position 6, and P (pro) at position 7. The variant may further comprise K (lys) at position 1, and/or N (asn) at position 3, and/or P (pro) at position 4. Additionally or alternatively, the variant may further comprise G (gly) at position 8, and/or P (pro) at position 9, and/or E (glu) at position 10. Additionally or alternatively, the variant may comprise one, two, three, four, five or six substituted residues. The substituted amino acid residue(s) may be at any one or more of positions 1, 3, 4, 8, 9, and/or 10 of **SEQ ID NO: 5.**

Alternatively, a variant of KVNPQ**G**P**G**P**E** (**SEQ ID NO: 5**) may comprise G (gly) at position 6, G (gly) at position 8, and E (glu) at position 10. The variant may further comprise N (asn) at position 3, and/or Q (gln) at position 5, and/or P (pro) at position 7. Additionally or alternatively, the variant may comprise one, two, three, four, five, six, or seven substituted residues. The substituted amino acid residue(s) may be at any one or more of positions 1, 2, 3, 4, 5, 7, and/or 9 of **SEQ ID NO: 5**.

Alternatively, a variant of KVNPQG**PG**P**E** (**SEQ ID NO: 5**) may comprise P (pro) at position 7, G (gly) at position 8, and E (glu) at position 10. The variant may further comprise Q (gln) at position 5, and/or G (gly) at position 6. Additionally or alternatively, the variant may comprise one, two, three, four, five, six, or seven substituted residues. The substituted amino acid residue(s) may be at any one or more of positions 1, 2, 3, 4, 5, 6, and/or 9 of **SEQ ID NO: 5.**

A variant of K**V**NP**QGP**GP (**SEQ ID NO: 6**) may comprise V (val) at position 2, Q (gln) at position 5, G (gly) at position 6, and and P (pro) at position 7. The variant may further comprise K (lys) at position 1, and/or N (asn) at position 3, and/or P (pro) at position 4. Additionally or alternatively, the variant may further comprise G (gly) at position 8, and/or P (pro) at position 9. Additionally or alternatively, the variant may comprise one, two, three, four, or five substituted residues. The substituted amino acid residue(s) may be at any one or more of positions 1, 3, 4, 8, and/or 9 of **SEQ ID NO: 6.**

A variant of KVNPQ**G**P**G**P (**SEQ ID NO: 6**) may comprise G (gly) at position 6, and G (gly) at position 8. The variant may further comprise N (asn) at position 3, and/or Q (gln) at position 5, and/or P (pro) at position 7. Additionally or alternatively, the variant may comprise one, two, three, four, five, six, or seven substituted residues. The substituted amino acid residue(s) may be at any one or more of positions 1, 2, 3, 4, 5, 7, and/or 9 of **SEQ ID NO: 6.**

A variant of KVNPQG**PG**P (**SEQ ID NO: 6**) may comprise P (pro) at position 7, and G (gly) at position 8. The variant may further comprise Q (gln) at position 5, and/or G (gly) at position 6. Additionally or alternatively, the variant may comprise one, two, three, four, five, six, or seven substituted residues. The substituted amino acid residue(s) may be at any one or more of positions 1, 2, 3, 4, 5, 6, and/or 9 **of SEQ ID NO: 6**.A variant of any one of the epitopes of the present invention as set forth in **SEQ ID NOs: 7-13** may comprise an amino acid substitution at any one or more position(s) of the epitope sequence. The amino acid substitution may be a conservative amino-acid substitution or a non-conservative amino acid substitution, as are known to those of ordinary skill in the art. The variants may comprise conservative substitution(s) only, non-conservative substitution(s) only, or a mixture of conservative substiution(s) and non-conservative substitution(s).

### - Fragments

Provided herein are fragments of the GPC-1 epitopes and variantsthereof..

In some embodiments, a fragment of an epitope of the present invention may comprise or consist of 4, 5, 6, 7, 8, 9, 10, 11, 12, 13, 14, 15, 16, 17, 18, 19 or 20 amino acids. Accordingly, a fragment of an epitope according to the present invention may comprise or consist of, for example, between 5 and 10, between 5 and 15, between 5 and 20, between 10 and 20, between 10 and 15, between 7 and 15, between 7 and 13, between 8 and 12, between 8 and 10, between 11 and 19, between 12 and 18, or between 13 and 17 amino acids in length. Generally, a fragment of a full GPC-1 epitope disclosed herein may possess similar or in some cases improved immunological properties compared to the full GPC-1 epitope.

In relation to the above embodiments, including those set out in **Tables 1-4** above, **Table 5** below provides suitable and non-limiting examples of the fragments referred to.

**Table 5: Non-limiting examples of GPC-1 epitope fragments according to the present invention**

| SEQ ID NO | SEQUENCE | EXEMPLARY FRAGMENTS |
|---|---|---|
| **SEQ ID NO: 1** | KVNPQGPGPEEK (or a variant thereof as disclosed herein) | residues 1-11, 1-10, 1-9, 1-8, 2-12,2-11, 2-10, 2-9, 2-8, 3-12, 3-11,3-10, 3-9, 3-8, 4-12, 4-11, 4-10, 4-9, or 4-8 |
| **SEQ ID NO: 2** | VNPQGPGPEEK (or a variant thereof as disclosed herein) | residues 1-10, 1-9, 1-8, 1-7, 2-11, 2-10, 2-9, 2-8, 2-7, 3-11, 3-10, 3-9, 3-8 or 3-7 |
| **SEQ ID NO: 3** | VNPQGPGPEE (or a variant thereof as disclosed herein) | residues 1-9, 1-8,1-7, 2-10,2-9,2-8, 2-7, 3-10, 3-9, 3-8 or 3-7 |
| **SEQ ID NO: 4** | NPQGPGPEE (or a variant thereof as disclosed herein) | residues 1-8, 1-7, 1-6, 2-9, 2-8, 2-7, 2-6, 3-9, 3-8, or 3-7 |
| **SEQ ID NO: 5** | KVNPQGPGPE (or a variant thereof as disclosed herein) | residues 1-9, 1-8, 1-7, 2-10, 2-9, 2-8, 2-7, 3-10, 3-9, 3-8 or 3-7 |
| **SEQ ID NO: 6** | KVNPQGPGP (or a variant thereof as disclosed herein) | residues 1-8, 1-7, 1-6, 2-9, 2-8, 2-7, 2-6, 3-9, 3-8, or 3-7 |
| **SEQ ID NO: 7** | TQNARAFRD (or a variant thereof as disclosed herein) | residues 1-8, 1-7, 1-6, 2-9, 2-8, 2-7, 3-9, 3-8, or 3-7 |
| **SEQ ID NO: 8** | TQNARA (or a variant thereof as disclosed herein) | residues 1-5, 1-4, 2-6, or 2-5 |
| **SEQ ID NO: 9** | ALSTASDDR (or a variant thereof as disclosed herein) | residues 1-8, 1-7, 1-6, 2-9, 2-8, 2-7, 3-9, 3-8, or 3-7 |
| **SEQ ID NO: 10** | PRERPP (or a variant thereof as disclosed herein) | residues 1-5, 1-4, 2-6, or 2-5 |
| **SEQ ID NO: 11** | QDASDDGSGS (or a variant thereof as disclosed herein) | residues 1-9, 1-8, 1-7, 2-10, 2-9, 2-8, 3-10, 3-9, or 3-8 |
| **SEQ ID NO: 12** | CGELYTQNARAFRDLCGNPKVNPQGPGPEEKR RRGC (or a variant thereof as disclosed herein) | residues 1-35, 1-34, 1-33, 1-32, 1-31, 1-30, 1-29, 1-28, 1-27, 1-26, 1-25, 2-36, 2-35, 2-34, 2-33, 2-32, 2-31, 2-30, 2-29, 2-28, 2-27, 2-26, 2-25, 3-36, 3-35, 3-34, 3-33, 3-32, 3-31, 3-30, 3-29, 3-28, 3-27, 3-26, 3-25, 4-36, 4-35, 4-34, 4-33, 4-32, 4-31, 4-30, 4-29, 4-28, 4-27, 4-26, 4-25, 5-36, 5-35, 5-34, 5-33, 5-32, 5-31, 5-30, 5-29, 5-28, 5-27, 5-26, 5-25, 6-36, 6-35, 6-34, 6-33, 6-32, 6-31, 6-30, 6-29, 6-28, 6-27, 6-26, 6-25, 7-36, 7-35, 7-34, 7-33, 7-32, 7-31, 7-30, 7-29, 7-28, 7-27, 7-26, 7-25, 8-36, 8-35, 8-34, 8-33, 8-32, 8-31, 8-30, 8-29, 8-28, 8-27, 8-26, 8-25, 9-36, 9-35, 9-34, 9-33, 9-32, 9-31, 9-30, 9-29, 9-28, 9-27, 9-26, 9-25, 10-36, 10-35, 10-34, 10-33, 10-32, 10-31, 10-30, 10-29, 10-28, 10-27, 10-26, or 10-25 |
| **SEQ ID NO: 13** | LGPECSRAVMK (or a variant thereof as disclosed herein) | residues 1-10, 1-9, 1-8, 1-7, 2-11, 2-10, 2-9, 2-8, 2-7, 3-11, 3-10, 3-9, 3-8 or 3-7 |

### - Fusion polypeptides

Certain embodiments of the present invention provide GPC-1 epitopes in the form of fusion polypeptides. For example, two or more full epitopes selected from **Table 1**, two or more epitope segments selected from **Table 2**, a combination of two or more full epitopes selected from **Table 3**, or a combination of a full epitope from **Table 1** and any one or more epitope segments selected from **Table 2**, may be linked together to form the fusion polypeptides.

The fusion polypeptides can be prepared using standard techniques known to those of ordinary skill in the art including, for example, chemical conjugation. The fusion polypeptides may also be expressed as a recombinant polypeptide in an expression system. For example, DNA sequences encoding polypeptide components of the fusion polypeptide may be assembled separately, and ligated into an appropriate expression vector. The 3' terminus of the DNA sequence encoding one polypeptide component can be ligated, with or without a peptide linker, to the 5' terminus of a DNA sequence encoding the second polypeptide component so that the reading frames of the sequences are in frame. This can allow translation into a single fusion polypeptide retaining the biological activity of both polypeptide components.

A linker sequence may be employed to separate first and second polypeptide components of the fusion protein by a distance sufficient to ensure that each polypeptide component folds into appropriate secondary and/or tertiary structures. Non-limiting examples of suitable linkers include peptides/polypeptides, alkyl chains or other convenient spacer molecules as known to those of ordinary skill in the art. Suitable peptide linker sequences may be selected according to factors including an ability to adopt a flexible extended conformation, an inability to adopt a secondary structure that could interact with functional epitope/s in polypeptide component/s of the fusion polypeptides, and/or a lack of hydrophobic and/or charged residues that may react with functional epitope/s in polypeptide component/s of the fusion polypeptide.

By way of non-limiting example only, the peptide linker sequences may contain Gly, Asn and/or Ser residues. Additionally or alternatively, other near neutral amino acids such as Thr and Ala may also be used in the peptide linker sequences. Further examples of amino acid sequences that may be usefully employed as linkers include those disclosed in US patent no. 4,935,233; US patent no. 4,751,180; Murphy et al., Proc. Natl. Acad. Sci. USA 83:8258-8262, 1986; and Maratea et al., Gene 40:39-46, 1985). The linker sequence may generally be from 1 to about 50 amino acids in length. Accordingly, the linker sequences may be 5, 10, 15, 20, 30, 40, between 10 and 50, between 10 and 40, between 10 and 30, between 20 and 30, between 1 and 5, or between 5 and 10 amino acids in length. Fusion polypeptides according to the present invention may not require a linker sequence if the first and second polypeptide components of the fusion polypeptide have non-essential N-terminal amino acid regions that can be used to separate the functional domains and prevent steric interference.

### - Polynucleotides, vectors and host cells

The present invention also provides polynucleotides encoding GPC-1 epitope/s of the present invention, segments of the GPC-1 epitopes, and fusion proteins comprising GPC-1 epitopes and/or segments thereof.

The polynucleotides may be cloned into a vector. The vector may comprise, for example, a DNA, RNA or complementary DNA (cDNA) sequence encoding the GPC-1 epitopes, GPC-1 epitope segment/s, and/or fusion proteins. The vector may be a plasmid vector, a viral vector, or any other suitable vehicle adapted for the insertion of foreign sequences, their introduction into cells and the expression of the introduced sequences. Typically the vector is an expression vector and may include expression control and processing sequences such as a promoter, an enhancer, ribosome binding sites, polyadenylation signals and transcription termination sequences.

Disclosed herein are alsohost cells transformed by such vectors. For example, the polynucleotides of the invention may be cloned into a vector which is transformed into a bacterial host cell, for example *E. coli.*

Methods for the construction of vectors and their transformation into host cells are generally known in the art, and described in, for example, Molecular Cloning: A Laboratory Manual (2nd ed., Cold Spring Harbor Laboratory Press, Plainview, New York, and, Ausubel F. M. et al. (Eds) Current Protocols in Molecular Biology (2007), John Wiley and Sons, Inc.

### - Production

GPC-1 epitopes of the present invention, segments of the GPC-1 epitopes, and fusion proteins comprising the GPC-1 epitopes and/or segments thereof can be manufactured according to standard methodologies well known to persons of ordinary skill in the art.

The epitopes, segments and fusion proteins may be prepared using any of a variety of well-known synthetic and/or recombinant techniques. Polypeptides may, for example, be generated by synthetic means using methodologies well known to those of ordinary skill in the art. In one non-limiting example, commercially available solid-phase techniques (e.g. the Merrifield solid-phase synthesis method) may be used in which amino acids are sequentially added to a growing amino acid chain (Merrifield, J. Am. Chem. Soc. 85:2149-2146, 1963). Equipment for automated synthesis of the epitopes, segments and fusion proteins disclosed herein is commercially available (e.g. Perkin Elmer/Applied BioSystems Division (Foster City, Calif.), and may be utilised according to the manufacturer's instructions.

Additionally or alternatively, the epitopes, segments and fusion proteins may be produced by any other method available to one of skill in the art. For example, recombinant means may be used in which nucleic acids encoding selected epitope/s, segment/s and/or fusion protein/s may be inserted into an expression vector using any of a variety of procedures known in the art (e.g. Sambrook et al., 1989, Molecular Cloning, A Laboratory Manual, 2d ed., Cold Spring Harbor Laboratory Press, Cold Spring Harbor, N.Y.; Sambrook and Russell, Molecular Cloning: A Laboratory Manual, 3rd edition (Jan. 15, 2001) Cold Spring Harbor Laboratory Press, ISBN: 0879695765; Ausubel et al., Current Protocols in Molecular Biology, Green Publishing Associates and Wiley Interscience, NY (1989)). Construction of suitable expression vectors containing nucleic acids encoding selected epitope/s, segment/s and/or fusion protein/s employs standard ligation techniques know to those of ordinary skill in the art.

The ligated nucleic acid sequences can be operably linked to suitable transcriptional or translational regulatory elements that facilitate expression of the epitope/s, segment/s and/or fusion protein/s. Regulatory elements responsible for the expression of proteins may be located 5' to the coding region for the polypeptide. Stop codons that end translation and transcription termination signals may be present 3' to the nucleic acid sequence encoding the epitope/s, segment/s and/or fusion protein/s. After construction of a nucleic acid encoding the polypeptide/s of interest with the operably linked regulatory elements, the resultant expression cassette can be introduced into a host cell and the encoded polypeptide/s can be expressed.

In accordance with the present invention, GPC-1 epitopes, segments of the GPC-1 epitopes, and fusion proteins comprising the GPC-1 epitopes and/or segments thereof, are "isolated". An "isolated" polypeptide is one that is removed from its original environment. For example, a naturally-occurring protein or polypeptide referred to herein is considered to be isolated if it is separated from some or all of the co-existing materials in the natural system. Isolated polypeptides referred to herein may also be purified. For example, the isolated polypeptides may be at least about 90% pure, at least about 95% pure or at least about 99% pure.

Polynucleotides and nucleic acids according to the present invention are generally known in the art, and are described, for example, in Ausubel F. M. et al. (Eds) Current Protocols in Molecular Biology (2007), John Wiley and Sons, Inc; Sambrook et al. (1989) Molecular Cloning: A Laboratory Manual (2nd ed., Cold Spring Harbor Laboratory Press, Plainview, New York; and Maniatis et al. Molecular Cloning (1982), 280-281. Polynucleotides may be prepared, for example, by chemical synthesis techniques, for example, the phosphodiester and phosphotriester methods (see for example Narang S. A. et al. (1979) Meth. Enzymol. 68:90; Brown, E. L. (1979) et al. Meth. Enzymol. 68:109; and U.S. Patent No. 4356270), the diethylphosphoramidite method (see Beaucage S.L et al. (1981) Tetrahedron Letters, 22:1859-1862). A method for synthesizing oligonucleotides on a modified solid support is described in U.S. Patent No. 4458066.

### - Supports

Epitopes and fusion polypeptides according to the present invention may be attached to a support. The support may, for example, be an insoluble material or a matrix which retains the epitope and excludes it from freely moving in the bulk of a reaction mixture. Suitable supports for immobilizing or localizing epitopes and fusion polypeptides are well known to those of ordinary skill in the art. The support can be selected from a wide variety of matrices, polymers, and the like in a variety of forms including beads convenient for use in microassays, plastic and glass plates with individual wells, as well as other materials compatible with the reaction conditions. In certain preferred embodiments, the support can be a plastic material, such as plastic beads or wafers, or that of the well or tube in which a particular assay (e.g. an ELISA) is conducted.

### Binding Entities

Disclosed herein are binding entities capable of binding specifically to the GPC-1 epitopes described herein.

The binding entity may be any molecule capable of binding specifically to a GPC-1 epitope as described herein. Non-limiting examples of binding entities include polypeptides, antibodies, antibody fragments, molecular imprints, lectins, and capture compounds. The binding entity may be an agent that can bind to a GPC-1 epitope of the present invention or alternatively a different region of GPC-1, and also modify a binding interaction between the epitope and another different binding entity such as, for example, an antibody as disclosed herein.

Binding entities such as antibodies capable of binding specifically to GPC-1 epitopes of the present invention can bind to a given target epitope, combination of epitope segments, or epitope combination preferentially over other non-target molecules. For example, if the binding entity ("molecule A") is capable of "binding specifically" or "specifically binding" to a given target GPC-1 epitope ("molecule B"), molecule A has the capacity to discriminate between molecule B and any other number of potential alternative binding partners. Accordingly, when exposed to a plurality of different but equally accessible molecules as potential binding partners, molecule A will selectively bind to molecule B and other alternative potential binding partners will remain substantially unbound by molecule A. In general, molecule A will preferentially bind to molecule B at least 10-fold, preferably 50-fold, more preferably 100-fold, and most preferably greater than 100-fold more frequently than other potential binding partners. Molecule A may be capable of binding to molecules that are not molecule B at a weak, yet detectable level. This is commonly known as background binding and is readily discernible from molecule B-specific binding, for example, by use of an appropriate control.

In the knowledge of the specific GPC-1 epitopes provided herein, persons of ordinary skill in the art can generate binding entities without inventive effort. For example, polyclonal and monoclonal antibody preparations that bind specifically to GPC-1 epitope/s of the present invention can be prepared using known techniques.

Any technique that provides for the production of antibody molecules by continuous cell lines in culture may be used in the preparation of monoclonal antibodies directed toward a target GPC-1 epitope. General methodology is described in Harlow and Lane (eds.), (1988), "Antibodies-A Laboratory Manual", Cold Spring Harbor Laboratory, N.Y. Specific methodologies include the hybridoma technique originally developed by Kohler et al., (1975), "Continuous cultures of fused cells secreting antibody of predefined specificity", Nature, 256:495-497, as well as the trioma technique, the human B-cell hybridoma technique (Kozbor et al., (1983), "The Production of Monoclonal Antibodies From Human Lymphocytes", Immunology Today, 4:72-79), and the EBV-hybridoma technique to produce human monoclonal antibodies (Cole et al., (1985), in "Monoclonal Antibodies and Cancer Therapy", pp. 77- 96, Alan R. Liss, Inc.). Immortal, antibody-producing cell lines can be created by techniques other than fusion, such as direct transformation of B lymphocytes with oncogenic DNA, or transfection with Epstein-Barr virus (see, for example, M. Schreier et al., (1980), "Hybridoma Techniques", Cold Spring Harbor Laboratory; Hammerling et al., (1981), "Monoclonal Antibodies and T-cell Hybridomas", Elsevier/North-Holland Biochemical Press, Amsterdam; Kennett et al., (1980), "Monoclonal Antibodies", Plenum Press).

In brief, a means of producing a hybridoma from which the monoclonal antibody is produced, a myeloma or other self-perpetuating cell line can be fused with lymphocytes obtained from the spleen of a mammal hyperimmunised with a recognition factor-binding portion thereof, or recognition factor, or an origin-specific DNA-binding portion thereof. Hybridomas producing a monoclonal antibody capable of binding specifically to a GPC-1 epitope of the present invention are identified by their ability to immunoreact with the epitope/s presented.

A monoclonal antibody useful in practicing the invention can be produced by initiating a monoclonal hybridoma culture comprising a nutrient medium containing a hybridoma that secretes antibody molecules of the appropriate antigen specificity. The culture is maintained under conditions and for a time period sufficient for the hybridoma to secrete the antibody molecules into the medium. The antibody-containing medium is then collected. The antibody molecules can then be further isolated by well known techniques.

Similarly, there are various procedures known in the art which may be used for the production of polyclonal antibodies. For the production of polyclonal antibodies against a given combination of GPC-1 epitopes, various host animals can be immunised by injection with the epitopes, including, but not limited to, rabbits, chickens, mice, rats, sheep, goats, etc. Further, the target molecule can be conjugated to an immunogenic carrier (e.g., bovine serum albumin (BSA) or keyhole limpet hemocyanin (KLH)). Also, various adjuvants may be used to increase the immunological response, including, but not limited to, Freund's (complete and incomplete), mineral gels such as aluminium hydroxide, surface active substances such as rysolecithin, pluronic polyols, polyanions, peptides, oil emulsions, keyhole limpet hemocyanins, dinitrophenol, and potentially useful human adjuvants such as BCG (bacille Calmette-Guerin) and Corynebacterium parvum.

Screening for the desired antibody can be accomplished by a variety of techniques known in the art. Suitable assays for immunospecific binding of antibodies include, but are not limited to, radioimmunoassays, ELISAs (enzyme-linked immunosorbent assay), sandwich immunoassays, immunoradiometric assays, gel diffusion precipitation reactions, immunodiffusion assays, *in situ* immunoassays, Western blots, precipitation reactions, agglutination assays, complement fixation assays, immunofluorescence assays, protein A assays, immunoelectrophoresis assays, and the like (see, for example, Ausubel et al., (1994), "Current Protocols in Molecular Biology", Vol. 1, John Wiley & Sons, Inc., New York). Antibody binding may be detected by virtue of a detectable label on the primary antibody. Alternatively, the antibody may be detected by virtue of its binding with a secondary antibody or reagent which is appropriately labelled. A variety of methods for the detection of binding in an immunoassay are known in the art and are included in the scope of the present invention.

The antibodies (or fragments thereof) raised against specific GPC-1 epitope/s of interest have binding affinity for the epitope/s. Preferably, the antibodies (or fragments thereof) have binding affinity or avidity greater than about 10⁵ M⁻¹, more preferably greater than about 10⁶ M⁻¹, still more preferably greater than about 10⁷ M⁻¹ and most preferably greater than about 10⁸M⁻¹.

In terms of obtaining a suitable amount of an antibody according to the present invention, one may manufacture the antibody(s) using batch fermentation with serum free medium. After fermentation the antibody may be purified via a multistep procedure incorporating chromatography and viral inactivation/removal steps. For instance, the antibody may be first separated by Protein A affinity chromatography and then treated with solvent/detergent to inactivate any lipid enveloped viruses. Further purification, typically by anion and cation exchange chromatography may be used to remove residual proteins, solvents/detergents and nucleic acids. The purified antibody may be further purified and formulated into 0.9% saline using gel filtration columns. The formulated bulk preparation may then be sterilised and viral filtered and dispensed.

Non-limiting examples of antibodies capable to binding to one or multiple GPC-1 epitopes of the present invention include:
(i) MIL38 antibody as deposited under the terms of the Budapest Treaty at Cellbank Australia at 214 Hawkesbury Road, Westmead, NSW 2145, Australia on 22^{nd} August 2014 under accession number CBA20140026;
(ii) anti-Glypican 1/GPC1 antibody (ab137604): a rabbit polyclonal (IgG) specific for human GPC-1 and commercially available from abcam® (see: http://www.abcam.com/glypican-1-gpc1-antibody-ab137604.html);
(iii) MAB2600| Anti-Glypican-1 Antibody, clone 4D1: a mouse monoclonal (IgGₖ) specific for human and rat GPC-1 and commercially available from Millipore (see: http://www.emdmillipore.com/AU/en/product/,MM_NF-MAB2600?cid=BI-XX-BRC-A-BIOC-ANTI-B033-1308);
(iv) goat anti-glypican 1 antibody (AA 24-530): a goat polyclonal specific for human GPC-1 and commercially available from LifeSpan BioSciences, Inc. (see https://www.lsbio.com/antibodies/anti-gpc1-antibody-glypican-antibody-aa24-530-icc-wb-western-flow-ls-c330760/341104).

### Compositions and Kits

Compositions and kits disclosed herein comprise:
- one or more GPC-1 epitope/s, variant/s or fragment/s thereof as described herein (e.g. **Table 1**); and/or
- one or more GPC-1 epitope segment/s, variant/s or fragment/s thereof as described herein (e.g. **Table 2**); and/or
- one or more GPC-1 epitope combinations as described herein (e.g. **Table 3**); and/or
- one or more fusion proteins comprising GPC-1 epitope/s, segment/s of GPC-1 epitopes, and/or variant/s or fragment/s thereof, as described herein (see subsection above entitled *"Fusion polypeptides* "); and/or
- one or more binding entities as described herein (see section above entitled *"Binding entities* ").

The compositions may additionally comprise an acceptable carrier, adjuvant and/or diluent. The carriers, diluents and adjuvants may be "acceptable" in terms of being compatible with the other ingredients of the composition, and/or "pharmaceutically acceptable" in generally being not deleterious to any recipient thereof. Suitable carriers, diluents and adjuvants are well known to those of ordinary skill in the art (see, for example, Gilman et al. (eds.) Goodman and Gilman's: the pharmacological basis of therapeutics, 8th Ed., Pergamon Press (1990); Remington's Pharmaceutical Sciences, Mack Publishing Co.: Easton, Pa., 17th Ed. (1985)). The compositions may, in some embodiments, be used for diagnostic or research purposes.

The kits may be fragmented or combined kits. A "fragmented kit" refers to a delivery system comprising two or more separate containers that each contain a sub portion of the total kit components. Any delivery system comprising two or more separate containers that each contain a sub portion of the total kit components are included within the meaning of the term fragmented kit. A "combined kit" refers to a delivery system containing all of the kit components in a single container (e.g. in a single box housing each of the desired components). The kits may, in some embodiments, be used for diagnostic or research purposes.

### Diagnostic Methods

GPC-1 epitopes of the present invention, segments of the GPC-1 epitopes, and fusion proteins comprising the GPC-1 epitopes and/or segments thereof, can be used in diagnostic methods. Specifically, the present inventors have discovered that glypican-1 is a new marker for prostate cancer (US provisional patent application no. 61/928/776 entitled *"Cell Surface Prostate Cancer Antigen for Diagnosis",* Walsh *et al. -* unpublished).

Accordingly, the present invention provides for methods for the diagnosis, prognosis, or likelihood of developing prostate cancer in subjects based on the detection of GPC-1 epitope/s, GPC-1 epitope segments, and/or variants and fragments of the epitopes/epitope segments as described herein. Additionally or alternatively, variants and fragments of the GPC-1 epitopes and/or GPC-1 epitope fragments may be detected in the diagnostic methods.

Generally, the methods comprise determining the level of GPC-1 epitope/s and/or GPC-1 epitope segments in a biological sample from a subject to be tested. Additionally or alternatively, the level of variant/s and fragment/s of the GPC-1 epitopes and/or segments thereof in the biological sample may also be determined when carrying out the methods. Non-limiting examples of GPC-1 epitopes, GPC-1 epitope segments, and variants and fragments include those referred to in the section entitled "Epitopes" (see in particular **Tables 1-3** and associated description of variants and fragments).

The methods may additionally comprise determining levels of prostate-specific antigen (PSA), also known as gamma-seminoprotein or kallikrein-3 (KLK3), in the biological sample, and optionally comparing the level of PSA detected with that of a control.

In some embodiments, the level of GPC-1 epitope/s, GPC-1 epitope segments, and/or variants and fragments of the epitopes/epitope segments detected in the subject's biological sample may be compared to levels determined to be present in a control sample. The levels in the control sample may be determined before, during or after determining the level present in the subject's biological sample. By way of non-limiting example, the levels present in the control sample may be determined based on those present in an equivalent biological sample from an individual or based on mean levels present in biological samples from a population of individuals. The individual or individuals may have been determined not to have cancer, and/or determined not to have prostate cancer. In general, detection of increased levels in the subject's biological sample compared to those of the control can be taken as indicative that the subject has prostate cancer, or an increased likelihood of developing prostate cancer. For example, an increase of at least 10%, at least 20%, at least 30%, at least 40%, at least 50%, or at least 60% may be indicative that the subject has prostate cancer, or an increased likelihood of developing prostate cancer. Alternatively, detection of equivalent or decreased levels in the subject's biological sample compared to those of the control can be taken as indicative that the subject does not have prostate cancer, or does not have an increased likelihood of developing prostate cancer.

In some embodiments, GPC-1 epitope/s, GPC-1 epitope segments, and/or variants and fragments of the epitopes/epitope segments as described herein are used as positive controls in the diagnostic methods of the present invention. For example, they may be used in a given assay to confirm that the assay as performed is capable of detecting the GPC-1 epitope/s, GPC-1 epitope segments, and/or variants and fragments.

In some embodiments, the diagnostic methods of the present invention utilise fusion protein/s of the present invention. Non-limiting examples of suitable fusion proteins are provided in the subsection entitled "Fusion proteins". The fusion protein/s may be used as a positive control in the detection methods.

The biological sample may be a tissue sample (e.g. a biopsy sample of prostate tissue) or a body fluid sample.

The body fluid sample may be a blood, serum, plasma or urine sample.

Non-limiting examples of prostate cancers that may be detected with the present invention include prostatic intraepithelial neoplasia, adenocarcinoma, leiomyosarcoma, and rhabdomyosarcoma.

Without limitation to specific detection methods and by way of non-limiting example only, the detection of the GPC-1 epitopes, GPC-1 epitope segments, and variants and fragments may be by way of standard assays known to those or ordinary skill in the art including, but not limited to Western blot analysis, Enzyme-linked immunosorbent assays (ELISAs), fluorescent activated cell sorting (FACS), a biofilm test, or an affinity ring test (see, for example, US application 2013/016,736). A binding entity according to the present invention may be used the assays.

In some embodiments, the binding entity is an antibody. In other embodiments, the biding entity is not an antibody
In some embodiments, the antibody is selected from any one or more of:
(i) MIL38 antibody as deposited under the terms of the Budapest Treaty at Cellbank Australia at 214 Hawkesbury Road, Westmead, NSW 2145, Australia on 22^{nd} August 2014 under accession number CBA20140026;
(ii) anti-Glypican 1/GPC1 antibody (ab137604): a rabbit polyclonal (IgG) specific for human GPC-1 and commercially available from abcam® (see: http://www.abcam.com/glypican-1-gpc1-antibody-ab137604.html);
(iii) MAB2600 Anti-Glypican-1 Antibody, clone 4D1: a mouse monoclonal (IgGₖ) specific for human and rat GPC-1 and commercially available from Millipore (see: http://www.emdmillipore.com/AU/en/product/,MM_NF-MAB2600?cid=BI-XX-BRC-A-BIOC-ANTI-B033-1308);
(iv) goat anti-glypican 1 antibody (AA 24-530): a goat polyclonal specific for human GPC-1 and commercially available from LifeSpan BioSciences, Inc. (see https://www.lsbio.com/antibodies/anti-gpc1-antibody-glypican-antibody-aa24-530-icc-wb-western-flow-ls-c330760/341104).

In some embodiments, the antibody is not selected from any one or more of:
(i) MIL38 antibody as deposited under the terms of the Budapest Treaty at Cellbank Australia at 214 Hawkesbury Road, Westmead, NSW 2145, Australia on 22^{nd} August 2014 under accession number CBA20140026;
(ii) anti-Glypican 1/GPC1 antibody (ab137604): a rabbit polyclonal (IgG) specific for human GPC-1 and commercially available from abcam® (see: http://www.abcam.com/glypican-1-gpc1-antibody-ab137604.html);
(iii) MAB2600 Anti-Glypican-1 Antibody, clone 4D1: a mouse monoclonal (IgGₖ) specific for human and rat GPC-1 and commercially available from Millipore (see: http://www.emdmillipore.com/AU/en/product/, MM_NF-MAB2600?cid=BI-XX-BRC-A-BIOC-ANTI-B033-1308);
(iv) goat anti-glypican 1 antibody (AA 24-530): a goat polyclonal specific for human GPC-1 and commercially available from LifeSpan BioSciences, Inc. (see https://www.lsbio.com/antibodies/anti-gpc1-antibody-glypican-antibody-aa24-530-icc-wb-western-flow-ls-c330760/341104).

In some embodiments, the binding entity is an antibody population comprising MIL38 antibody (CBA20140026) and not comprising an anti-glypican 1 (GPC-1) antibody capable of binding to an epitope comprising an amino acid sequence selected from any one or a plurality of: TQNARA (**SEQ ID NO: 8**), ALSTASDDR (**SEQ ID NO:** 9), PRERPP (**SEQ ID NO: 10**), QDASDDGSGS (**SEQ ID NO: 11**), LGPECSRAVMK (**SEQ ID NO: 13**), and TQNARAFRD (**SEQ ID NO: 7**).

### Examples

The present invention will now be described with reference to specific Examples, which should not be construed as in any way limiting.

### Example 1: identification and characterisation of a glypican-1 epitope bound by murine anti-human glypican 1 (MIL38-AM4)

### 1.1 Materials and Methods

Empirical sets spanning the reference sequence with different conformational constraints were made, and the antibody was probed on the peptide array.

### - Antibodies

Murine anti human glypican 1 monoclonal antibody MIL38-AM4 was provided as set out in **Table 6** below.

**Table 6: description of antibody used**

| **Name** | **Origin** | **Concentration** | **Storage** | **Status** |
|---|---|---|---|---|
| **MIL38-AM4*** | Mouse | 4.6mg/ml(100µl) | -20 °C/ 73 | OK |

| | | | | |
|---|---|---|---|---|
| ** produced by hybridoma cells as deposited at Cellbank Australia under accession number CBA20140026* | | | | |

### - Peptides

The following human glypican-1 (GPC-1) sequence was used as a basis to generate a library of structured peptides.
**P35052** (GPC1_HUMAN)

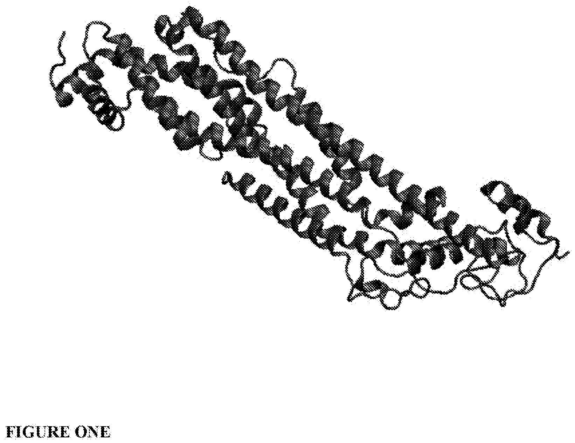

**Figure 1A** shows the rendering of chain B as present under Protein Data Bank identifier (PBD ID) 4AD7 (http://www.ebi.ac.uk/pdbsum/4AD7).

### - Chemically Linked Peptides on Scaffolds (CLIPS) technology

The following provides description of general principles of the CLIPS technology utilised.

CLIPS technology structurally fixes peptides into defined three-dimensional structures. This results in functional mimics of even the most complex binding sites. CLIPS technology is now routinely used to shape peptide libraries into single, double or triple looped structures as well as sheet- and helix-like folds.

The CLIPS reaction takes place between bromo groups of the CLIPS scaffold and thiol sidechains of cysteines. The reaction is fast and specific under mild conditions. Using this chemistry, native protein sequences are transformed into CLIPS constructs with a range of structures including single T2 loops, T3 double loops, conjugated T2+T3 loops, stabilized beta sheets, and stabilized alpha helixes (Timmerman et al., J. Mol. Recognit. 2007; 20: 283-29).

CLIPS library screening starts with the conversion of the target protein into a library of up to 10,000 overlapping peptide constructs, using a combinatorial matrix design. On a solid carrier, a matrix of linear peptides is synthesized, which are subsequently shaped into spatially defined CLIPS constructs. Constructs representing both parts of the discontinuous epitope in the correct conformation bind the antibody with high affinity, which is detected and quantified. Constructs presenting the incomplete epitope bind the antibody with lower affinity, whereas constructs not containing the epitope do not bind at all. Affinity information is used in iterative screens to define the sequence and conformation of epitopes in detail.

The target protein containing a discontinuous conformational epitope is converted into a matrix library. Combinatorial peptides are synthesized on a proprietary minicard and chemically converted into spatially defined CLIPS constructs. Binding of antibodies is quantified.

### - Peptide synthesis

To reconstruct discontinuous epitopes of the target molecule a library of structured peptides was synthesized. This was done using Chemically Linked Peptides on Scaffolds (CLIPS) technology. CLIPS technology allowed the generation of structured peptides in single loops, double-loops, triple loops, sheet-like folds, helix-like folds and combinations thereof. CLIPS templates were coupled to cysteine residues. The side-chains of multiple cysteines in the peptides were coupled to one or two CLIPS templates. For example, a 0.5 mM solution of the T2 CLIPS template 1,3-bis (bromomethyl) benzene was dissolved in ammonium bicarbonate (20 mM, pH 7.9)/acetonitrile (1:1(v/v)). This solution was added onto the peptide arrays. The CLIPS template bound to side-chains of two cysteines as present in the solid-phase bound peptides of the peptide-arrays (455 wells plate with 3 µl wells). The peptide arrays were gently shaken in the solution for 30 to 60 minutes while completely covered in solution. Finally, the peptide arrays were washed extensively with excess of H₂O and sonicated in disrupt-buffer containing 1 percent SDS/0.1 percent betamercaptoethanol in PBS (pH 7.2) at 70°C for 30 minutes, followed by sonication in H₂O for another 45 minutes. The T3 CLIPS carrying peptides were made in a similar way but with three cysteines.

Linear and CLIPS peptides were chemicall synthesized according to the following designs:

**Set 1**

| | |
|---|---|
| Label | lin |
| Mimic Type | Linear peptides |
| Description | Linear peptides of length 15 with an overlap of 14 that cover reference sequence (**SEQ ID NO: 14**). |

**Set 2**

| | |
|---|---|
| Label | loop |
| Mimic Type | Loop shaped peptides |
| Description | Peptides of length 17, with cysteine residues at positions 1 and 17. Positions 2 - 16 contain the linear 15mers of Set 1 in which cysteines are replaced by alanine. After synthesis, peptides are constrained by a P2 CLIPS™ linker to constrain the shape. |

**Set 3**

| | |
|---|---|
| Label | hel_i3 |
| Mimic Type | Helical peptides |
| Description | Peptides of length 15, with an overlap of 14 derived from the reference sequence (**SEQ ID NO: 14**). Cysteine residues were replaced by Alanine. Then positions 3 and 7 were replaced by Cysteine, which were connected by a P2 CLIPS™. This i, i+4 connection ('stapling') induced helix nucleation in sequences that were prone to helical folding. |

**Set 4**

| | |
|---|---|
| Label | hel_i7 |
| Mimic Type | Helical peptides |
| Description | Peptides of length 15, with an overlap of 14 derived from the reference sequence (**SEQ ID NO: 14**). Cysteine residues were replaced by Alanine. Then positions 3 and 11 were replaced by Cysteine, which were connected by a P2 CLIPS™. This i, i+7 connection ('stapling') induced helix nucleation in sequences that were prone to helical folding. |

**Set 5**

| | |
|---|---|
| Label | sheet |
| Mimic Type | Sheet shaped peptides |
| Description | Peptides of length 15, with an overlap of 14 derived from the reference sequence (**SEQ ID NO: 14**). Cysteine residues were replaced by Alanine. Then positions 6 and 9 were replaced by Cysteine, which were connected by a P2 CLIPS™. In peptides prone to β sheet folding this shape was stabilized. |

### - ELISA screening

The binding of antibody to each of the synthesized peptides was tested in a PEPSCAN-based ELISA. The peptide arrays were incubated with primary antibody solution (overnight at 4°C). After washing, the peptide arrays were incubated with a 1/1000 dilution of an antibody peroxidase conjugate (SBA, cat.nr. 2010-05) for one hour at 25°C. After washing, the peroxidase substrate 2,2'-azino-di-3-ethylbenzthiazoline sulfonate (ABTS) and 2 µl/ml of 3 percent H₂O₂ were added. After one hour, the color development was measured. The color development was quantified with a charge coupled device (CCD) - camera and an image processing system.

### - Data processing

The values obtained from the CCD camera ranged from 0 to 3000 mAU, similar to a standard 96-well plate ELISA-reader. The results were quantified and stored in the Peplab database. Occasionally a well contained an air-bubble resulting in a false-positive value. The cards were manually inspected and any values caused by an air-bubble were scored as 0.

### - Synthesis quality control

To verify the quality of the synthesized peptides, a separate set of positive and negative control peptides was synthesized in parallel. These were screened with antibody 57.9 (*ref.* Posthumus et al., J. Virology, 1990, 64:3304-3309).

### - Screening details

**Table 7** summarises antibody binding conditions. For the Pepscan Buffer and Preconditioning (SQ), the numbers indicate the relative amount of competing protein (a combination of horse serum and ovalbumin).

**Table 7: screening conditions**

| | **dilution** | **samplebuffer** | **preconditioning** |
|---|---|---|---|
| **serum MIL38-AM4*** | 1µg/ml | 50%SQ | 50%SQ |
| **MIL38-AM4*** | 10µg/ml | 50%SQ | 50%SQ |

| | | | |
|---|---|---|---|
| ** produced by hybridoma cells as deposited at Cellbank Australia under accession number CBA20140026* | | | |

### 1.2 Results

### - Primary experimental results and signal to noise ratio determination

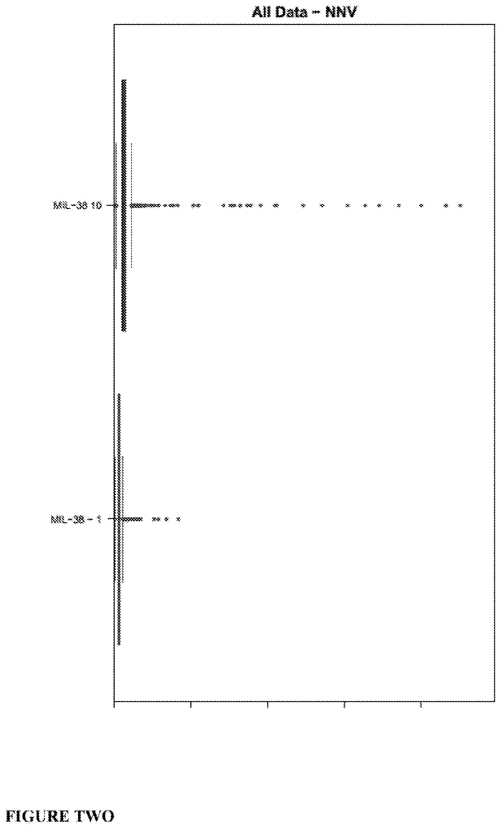

A graphical overview of the complete dataset of raw ELISA results generated by the screening is shown in **Figure 2****.** Here a box plot depicts each dataset and indicates the average ELISA signal, the distribution and the outliers within each dataset. Depending on experiment conditions (amount of antibody, blocking strength etc) different distributions of ELISA data were obtained. The data enabled identification of an epitope.

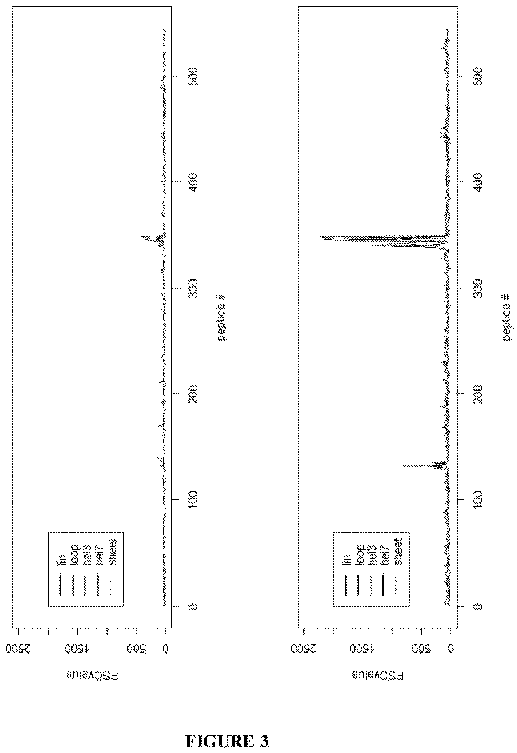

The array was incubated with MIL38-AM4 at dilutions of 1 µg/ml and 10 µg/ml, under normal stringency conditions. A concentration dependent response was observed (**Figures 3A and 3B**). At 10 µg/ml saturation was not observed, indicating that none of the peptides represented the full epitope. Two clusters of responses that are not adjacent in the primary structure were identified. Two common cores emerged: ₁₃₅TQNARAFRD₁₄₃ (**SEQ ID NO: 7**) and ₃₄₈VNPQGPGPEEK₃₅₈ (**SEQ ID NO: 2**), with the latter stretch clearly dominant with respect to the former.

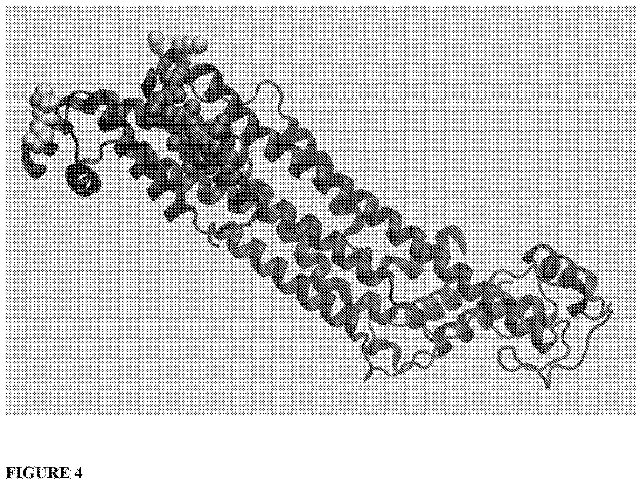

In the three-dimensional (3D) model derived from Protein Data Bank identifier (PBD ID) 4AD7 (http://www.ebi.ac.uk/pdbsum/4AD7) (**Figure 4**), the stretch ₃₄₈VNPQGPGPEEK₃₅₈ (**SEQ ID NO: 2**) was not resolved, nor was it in another publicly available pdb coordinate file (4ACR, 4BWE), indicating that it probably is flexible. The stretch ₁₃₅TQNARAFRD₁₄₃ (**SEQ ID NO: 7**) lies adjacent to this missing loop, as is evident from the positions of V348 and G362, which are resolved in the 3D model.

It is common to see binding to incomplete mimics that partially fulfill antibody requirements similar to the binding that we observe for MIL38-AM4 to the peptides in this array. We postulate a discontinuous epitope, consisting of major contributions from the flexible loop ₃₄₈VNPQGPGPEEK₃₅₈ (**SEQ ID NO: 2**), and minor contributions from (residues of) the helix ₁₃₅TQNARAFRD₁₄₃ (**SEQ ID NO: 7**).

### 1.3 Summary and Discussion

This study aimed to map the epitope of murine anti human glypican 1 (MIL38-AM4). Empirical sets spanning the reference sequence with different conformational constraints were made, and the antibody was probed on the peptide array. Significant binding indicated a discontinuous epitope, consisting of major contributions from the flexible loop ₃₄₈VNPQGPGPEEK₃₅₈ (**SEQ ID NO: 2**), and minor contributions from (residues of) the helix ₁₃₅TQNARAFRD₁₄₃ (**SEQ ID NO: 7**).

### Example 2: identification and characterisation of additional glypican-1 epitopes bound by other anti-glypican-1 antibodies

### 2.1 Materials and Methods

**Table 8** below provides information on the antibodies used in this study. The first three antibodies listed are commercially available.

**Table 8: description of antibodies used**

| **Name** | **Origin** | **Concentration** | **Location** | **Status** |
|---|---|---|---|---|
| **Anti-Glypican 1 (GPC1) (AA 24-530)^** | goat | 1mg/ml | 4 °C/ 11 | OK |
| **Anti Glypican 1/GPC1 antibody (ab137604)*** | rabbit | 0.97mg/ml | -20 °C/ 73 | OK |
| **Mab2600 Merck Millipore^{†} (anti-glypican-1 Ab, clone 4D1)** | mouse | 1mg/ml | 4 °C/ 11 | OK |
| **MIL38-AM3^{§}** | mouse | 1.25mg/ml | -20 °C/ 73 | used |

| | | | | |
|---|---|---|---|---|
| ^ *Commercially available from: antibodies-online (http:*//*www.antibodies-online.com); Lifespan Biosciences, Inc. (https:*//*www.lsbio.com)* ** Commercially available from abeam® (http:*//*www.abcam.com)* *† Commercially available from Merck Millipore (http:llwww.emdmillipore.com)* *§ One of two distinct antibody populations produced by mixed hybridoma deposited at the American Tissue Type Culture Collection (ATCC) under accession number HB11785* | | | | |

### - Peptides

The human glypican-1 (GPC-1) sequence defined by **SEQ ID NO: 14** was used as a basis to generate a library of structured peptides.

**Figure 1A** shows the rendering of chain B as present under Protein Data Bank identifier (PBD ID) 4AD7 (http://www.ebi.ac.uk/pdbsum/4AD7).

### - Chemically Linked Peptides on Scaffolds (CLIPS) technology

The general principles of the CLIPS technology utilised in these experiments is set out in Example 1 above.

### - Peptide synthesis

Peptide synthesis was performed using the methods referred in Example 1. Chemically synthesized linear and CLIPS peptides were synthesized according to the designs shown in Example 1 (sets 1-5).

### - ELISA screening

The binding of antibody to each of the synthesized peptides was tested in a PEPSCAN-based ELISA, as set out in Example 1.

### - Data processing and synthesis quality control

Data processing and synthesis quality control was performed as per Example 1.

### - Screening details

**Table 9** summarises antibody binding conditions. For the Pepscan Buffer and Preconditioning (SQ), the numbers indicate the relative amount of competing protein (a combination of horse serum and ovalbumin). P/Tw (PBS-Tween) was also used to reduce stringency of binding.

**Table 9: screening conditions**

| **Sample** | **Concentration** | **Sample buffer** | **Precoat** |
|---|---|---|---|
| **Anti Glypican 1/GPC1 antibody (ab137604)*** | 1/1000 | SQ | SQ |
| **Anti Glypican 1/GPC1 antibody (ab137604)*** | 1/10000 | SQ | SQ |
| **Anti-Glypican 1 (GPC1) (AA 24-530)** | 1 µg/ml | SQ | SQ |
| **Mab 2600 Merck Millipore (anti-glypican-1 Ab, clone 4D1)** | 10ng/ml | SQ | SQ |
| **Anti-Glypican 1 (GPC1) (AA 24-530)** | 0.01 µg/ml | SQ | SQ |
| **MIL38-AM3** | 1 µg/ml | 10%SQ | 10%SQ |
| **MIL38-AM3** | 10ug/ml | 10%SQ | 10%SQ |
| **MIL38-AM3** | 2.5µg/ml | P/Tw | 0.1%SQ |

### 2.2 Results

### - Primary experimental results and signal to noise ratio determination

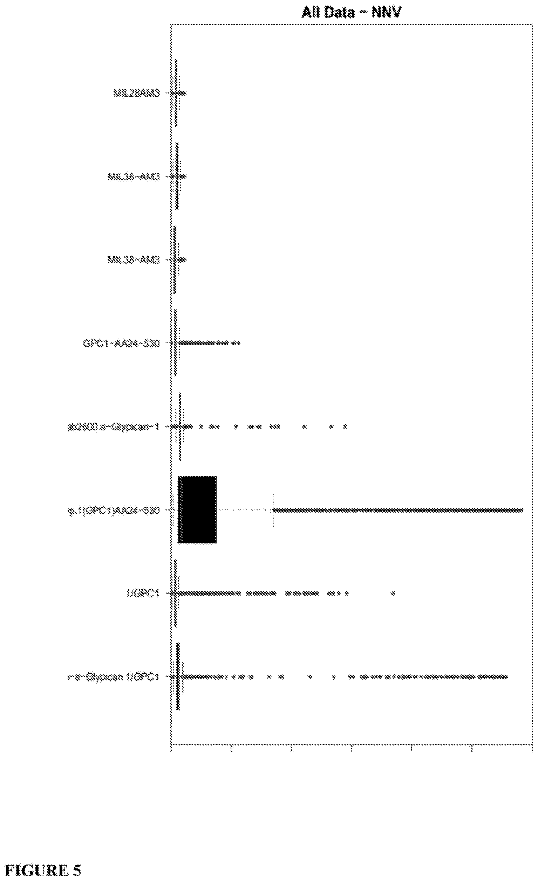

A graphical overview of the complete dataset of raw ELISA results generated by the screening is shown in **Figure 5**. Here a box plot depicts each dataset and indicates the average ELISA signal, the distribution and the outliers within each dataset. Depending on experiment conditions (amount of antibody, blocking strength etc) different distributions of ELISA data are obtained. The data enabled identification of an epitope for the monoclonal and polyclonal sera, but not for MIL38-AM3.

### - MIL38-AM3

Even when tested at high concentrations (10µg/ml) and at reduced stringency (PBS-Tween) MIL38-AM3 could not be detected. The sample was also tried with a different secondary antibody (Sheep Anti Mouse IgG HRP; GE Healthcare, NXA931), but again no result was obtained.

As a confirmation we tried a direct ELISA, in which MIL38-AM3 was coated onto the plate. Using Rabbit Anti Mouse IgG - HRP (Southern Biotech) the antibody could not be detected.

### - Rabbit Anti Glypican1/GPC1 antibody ab137604

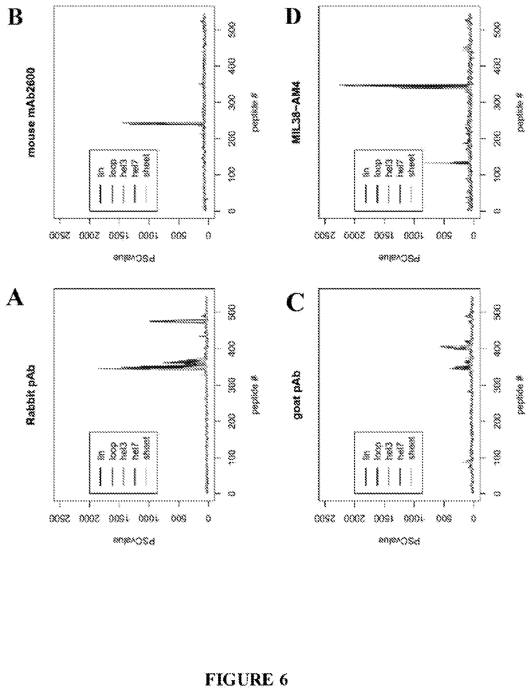

Rabbit anti GPC1 yields 3 main signals, as can be seen in the corresponding panel in **Figure 6****.** These signals correspond to the stretches ₃₄₈VNPQGPGPEEK₃₅₈ **(SEQ ID NO: 2),** ₃₆₆PRERPP₃₇₁ **(SEQ ID NO: 10),** and ₄₇₈QDASDDGSGS₄₈₇ **(SEQ ID NO: 11).**

### - Mouse mAb 2600 (Millipore)

Mouse antibody 2600 recognizes one clear peak in all peptide sets, sharing the common core ₂₄₂LGPECSRAVMK₂₅₂ **(SEQ ID NO: 13).** This can be seen in the corresponding panel in **Figure 6****.**

### - Goat anti-GPC-1 (GPC1)

Goat anti-GPC-1 yields 2 main signals, as can be seen in the corresponding panel in **Figure 6****.** These signals correspond to the stretches ₃₄₈VNPQGPGPEEK₃₅₈ **(SEQ ID NO: 2),** and ₄₀₈ALSTASDDR₄₁₄ **(SEQ ID NO: 9).**

### 2.3 Summary and Discussion

This study aimed to profile three commercially available anti-Glypican 1 antibodies and an extra mAb (MIL38-AM3) on the same arrays. The antibodies were probed on an existing peptide array as set out in Example 1. The commercially available anti GPC-1 antibodies could all be mapped using these arrays. Antibody MIL38-AM3 proved refractory to mapping, either due to the discontinuous nature of the epitope or to sample degradation, and did not yield signal on any of the arrays.

Rabbit anti GPC-1 recognizes at least three stretches in glypican 1, ₃₄₈VNPQGPGPEEK₃₅₈ **(SEQ ID NO: 2),** ₃₆₆PRERPP₃₇₁ **(SEQ ID NO: 10),** and _{478Q}DASDDGSGS₄₈₇ **(SEQ ID NO: 11).** Two of these stretches are resolved in coordinate file 4AD7.pbd, as depicted in **Figure 7A****.** Since this is a polyclonal antibody preparation, it cannot be assessed whether the epitopes are linear or are part of a complex epitope.

Mouse anti glypican mab 2600 recognizes the stretch ₂₄₂LGPECSRAVMK₂₅₂ **(SEQ ID NO: 13)** in all peptide sets of the array. The localization of epitope in coordinate file 4AD7.pdb is depicted in **Figure 7B****.**

Goat anti glypican 1 recognizes at least two stretches in glypican 1, ₃₄₈VNPQGPGPEEK₃₅₈ **(SEQ ID NO: 2),** and ₄₀₈ALSTASDDR₄₁₄ **(SEQ ID NO: 9).** Since this is a polyclonal antibody preparation, it cannot be assessed whether the epitopes are linear or are part of a complex epitope. Neither stretch is resolved in the available coordinate file.

The rabbit and goat polyclonal preparations both recognize a stretch ₃₄₈VNPQGPGPEEK₃₅₈ **(SEQ ID NO: 2)** in Glypican 1, which also forms part of the (likely discontinuous) binding site of Mab MIL38-AM4. Both polyclonal preparations also recognize additional epitopes on Glypican 1, but it cannot be assessed if these epitopes form a discontinuous epitope, or are manifestations of the polyclonal nature of the sample. Neither pAb recognizes the stretch ₁₃₅TQNARA₁₄₀, which is thought to contribute to MIL38-AM4 binding.

Mouse Mab 2600 recognizes an epitope that is not shared by any other anti-GPC-1 antibody tested thus far.

### Example 3: identification and characterisation of additional glypican-1 epitopes bound by other anti-glypican-1 antibodies

### 3.1 Materials and Methods

**Table 10** below provides information on the antibodies used in this study. Three anti-glypican 1 antibodies were used, each having been used in earlier experiments as described in Example 1 and/or Example 2 above.

**Table 10: description of antibodies used**

| **Name** | **Origin** | **Concentration** | **Location** | **Status** |
|---|---|---|---|---|
| **Anti-Glypican 1 (GPC1) (AA 24-530)^** | goat | 1 mg/ml | 4 °C/ 11 | OK |
| **Anti Glypican 1/GPC1 antibody (ab137604)*** | rabbit | 0.97mg/ml | -20 °C/ 73 | OK |
| **MIL38-AM4^{†}** | mouse | 4.6 mg/ml | -20 °C/ 73 | OK |

| | | | | |
|---|---|---|---|---|
| ^ *Commercially available from: antibodies-online (http:*//*www.antibodies-online.com); Lifespan Biosciences, Inc. (https:*//*www.lsbio.com)* ** Commercially available from abeam® (http:*//*www.abcam.com)* *† Produced by hybridoma cells as deposited at Cellbank Australia under accession number CBA20140026* | | | | |

### - Peptides

The human glypican-1 (GPC-1) sequence on which this study was based is defined in **SEQ ID NO: 14.** The following sequences of residues were used:
GPC1_residues #344-366 GNPKVNPQGPGPEEKRRRGKLAP **(SEQ ID NO: 15)**
GPC1_residues #131-149 GELYTQNARAFRDLYSELR **(SEQ ID NO**: **16)**

### - Peptide synthesis

Peptide synthesis was performed using the methods referred in Example 1. Chemically synthesized linear and CLIPS peptides were synthesized according to the designs shown below:

**Set 1**

| Mimic Type | Discontinuous epitope mimics |
|---|---|
| Label | MAT.A, MAT.B |
| Description | Constrained peptide mimics of varying length. From the two starting sequences (**SEQ ID NO: 15** and **SEQ ID NO: 16**) all 10 to 22, and 10 to 18 mer peptides with stepsize 4 were made, and these were been paired. At the termini and in between the two peptides cysteines were placed. These were linked by a T3 CLIPS. |

**Set 2**

| Mimic Type | Linear peptides |
|---|---|
| Label | RN.PKVNPQGPGPEEKRR (**SEQ ID NO: 17**) |
| Description | Substitution analysis, starting from the base sequence PKVNPQGPGPEEKRR (**SEQ ID NO: 18**), all individual amino acids were replaced by all naturally occurring amino acids, except cysteine. |

**Set 3**

| Mimic Type | Constrained peptides. |
|---|---|
| Label | RN.PKVNPQGPGPEEKRR LOOP **(SEQ ID NO: 17),** |
| | RN.ELCTQNCRAFRDLYS_heli3 **(SEQ ID NO: 19)** |
| | RN.ELCTQNCRAFRDLYS_LOOP **(SEQ ID NO: 19)** |
| Description | Substitution analyses, starting from the base sequences as indicated in the label names, all individual amino acids were replaced by all naturally occurring amino acids, except cysteine. |

### - ELISA screening

The binding of antibody to each of the synthesized peptides was tested in a PEPSCAN-based ELISA, as set out in Example 1.

### - Data processing and synthesis quality control

Data processing and synthesis quality control was performed as per Example 1.

### - Heat map analysis

A brief overview of the heat map methodology used is set out below.

A heat map is a graphical representation of data where the values taken by a variable in a two-dimensional map are represented as colours. For double-looped CLIPS peptides, such a two-dimensional map can be derived from the independent sequences of the first and second loops. For example, the sequences of a given series of 16 CLIPS peptides are effectively permutations of 4 unique sub-sequences in loop 1 and 4 unique sub-sequences in loop 2. Thus, the observed ELISA data can be plotted in a 4x4 matrix, where each X coordinate corresponds to the sequence of the first loop, and each Y coordinate corresponds to the sequence of the second loop.

To further facilitate the visualization, ELISA values can be replaced with a continuous gradient of shading. In this case, extremely low values are light coloured, extremely high values are darker coloured, and average values are black coloured. When this gradient map is applied to a data matrix, a shaded heat map is obtained.

### - Screening details

**Table 11** summarises antibody binding conditions. For the Pepscan Buffer and Preconditioning (SQ), the numbers indicate the relative amount of competing protein (a combination of horse serum and ovalbumin).

**Table 11: screening conditions**

| **serum** | **dilution** | **samplebuffer** | **preconditioning** |
|---|---|---|---|
| **Anti-Glypican 1 (GPC1) (AA 24-530)** | 10 ng/ml | SQ | SQ |
| **Anti Glypican 1/GPC1 antibody (ab137604)** | 1/2500 | SQ | SQ |
| **MIL-38 AM4** | 10 µg/ml | 10%SQ | 50%SQ |

### 3.2 Results

### - Primary experimental results and signal to noise ratio determination

A graphical overview of the complete dataset of raw ELISA results generated by the screening is shown in **Figure 8****.** Here a box plot depicts each dataset and indicates the average ELISA signal, the distribution and the outliers within each dataset. Depending on experiment conditions (amount of antibody, blocking strength etc) different distributions of ELISA data are obtained.

### - Rabbit polyclonal Ab 137604

In Example 2 the stretch ₃₄₈VNPQGPGPEEK₃₅₈ **(SEQ ID NO: 2)** was found to suffice for binding some IgG from rabbit polyclonal Ab 137604. In this study all constructs containing ₃₄₈VNPQGPGPEE₃₅₇ **(SEQ ID NO: 3)** again were bound by IgG from this sample at 1/2500 dilution. In the matrices of Set1 there is no augmentation of signal in specific constructs.

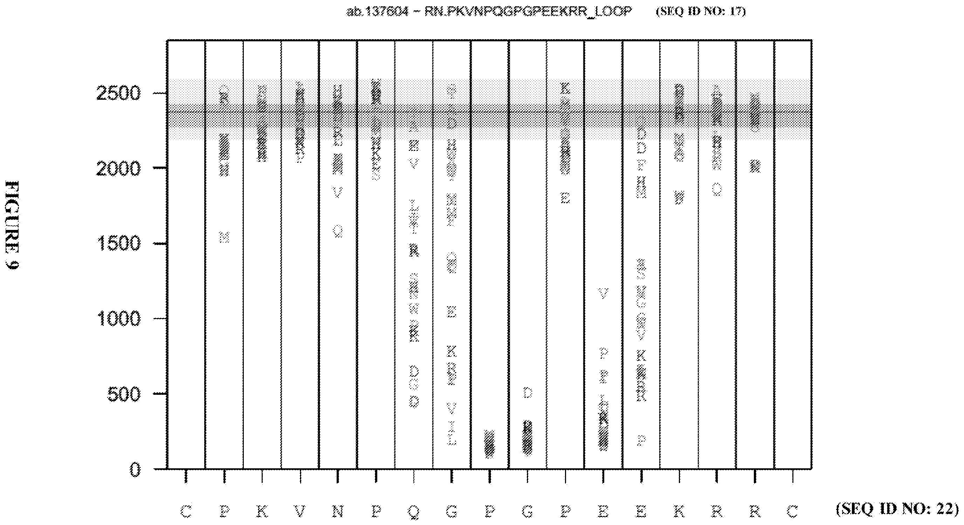

From the substitution analysis in **Figure 9** it can be seen that residues P353, G354, and E356 are essential. Limited substitutions are allowed at positions Q351, G352, and E357.

### - Goat polyclonal antiGPC-1

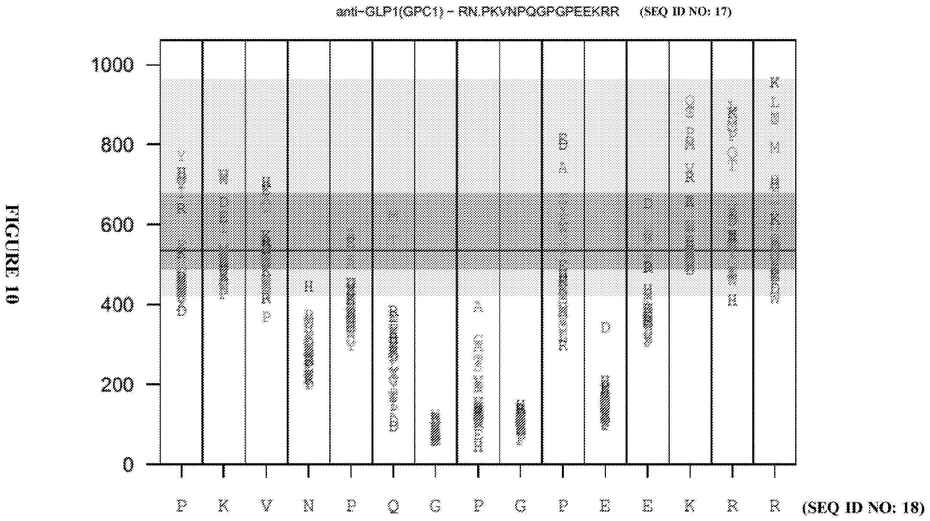

In Example 2 the stretch ₃₄₈VNPQGPGPEEK₃₅₈ **(SEQ ID NO: 2)** also sufficed to bind some IgG from goat polyclonal antiGPC-1. This antibody recognizes the same loop as the rabbit polyclonal, but does so with a slightly different fine specificity. In the Letterplot of **Figure 10** it can be seen that the most critical residues are G352, G354, and E356, and that limited substitution is allowed on positions N349, Q351, and P353. In the matrices of Set1 augmentation of signal is seen. Although the stretch around residues 140 - 149 was not implied in the primary mapping of this antibody, distinct constructs including this stretch are better baits for binding by some IgG in this polyclone.

### - Antibody MIL38-AM4

In Examples 1 and 2 it was established that MIL38-AM4 binds glypican on stretch ₃₄₈VNPQGPGPEEK₃₅₈ **(SEQ ID NO: 2),** and also binds to the stretch ₁₃₅TQNARA₁₄₀ **(SEQ ID NO: 8),** which was taken as an indication for a discontinuous epitope.

The looped constructs containing the main stretch pinpoint the residues that are most critical to binding. From **Figure 10** it can be seen that residues V348, Q351, G352, and P353 do not tolerate replacement, with significant requirement for K347, N349 and P350, and to a lesser extent from G354, P355, and E356.

Optimization of a mimic for recognition by MIL38-AM4 by adding residues from the range 135 - 143 to the main loop matrices of Set1.

The requirement for V348 and surrounding residues was again evident in these series. There was additional binding in the matrix sets culminating in optimal signal for T3 constrained CGELYTQNARAFRDLCGNPKVNPQGPGPEEKRRRGC **(SEQ ID NO: 12) (****Figure 12****).**

### - Similarities and dissimilarities

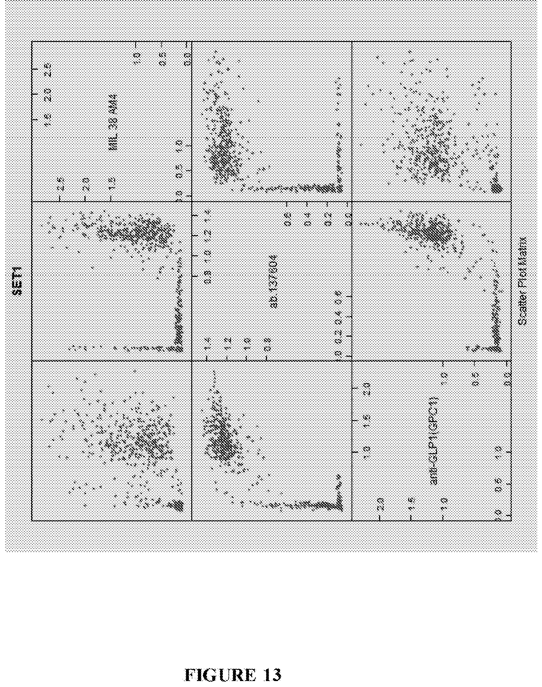

The antibodies bind to or not bind to similar constructs, as can be seen in the scatter plots of **Figure 13****.** However, many constructs are exclusively seen by one of the preparations (points along the axes, or in the lower right hand and upper left hand corners).

### - Conclusions

In follow-up to Examples 1 and 2, the conformational epitope of antibody MIL 38-AM4 was profiled. Polyclonal antibodies AB137604 (Rabbit) and anti GPC-1 24-530 (Goat) were found to recognize a similar epitope. These were contrasted and compared on the same arrays.

The two leads obtained in Example 1 that point to a discontinuous epitope for MIL 38 - AM4 were used to generate a matrix array in which the loops have different lengths. In addition, full substitution analyses of the individual lead sequences were made. All arrays were probed with the three antibodies listed above.

For recognition of glypican 1, all antibodies investigated in this study bind to an epitope that exclusively or mainly consists of the flexible loop between residues 347 and 358. However their fine specificities differ, which may have implications for their functional properties, which in turn may influence selectivity *in vivo* or applicability in discriminative tests.

The epitope of (some IgG species present in) rabbit polyclonal Ab 137604 is the linear stretch ₃₄₈VNPQGPGPEE₃₅₇ **(SEQ ID NO: 3).** There is no indication for the presence of antibodies that recognize a conformational epitope.

The same flexible loop is also seen by (some IgG species in) goat polyclonal anti GPC-1. There is some preference for structured mimics, although this is not major. It may well be that not all antibodies in this preparation see the flexible loop in the same manner. Monoclonal antibody MIL38-AM4 mainly binds glypican 1 on the loop between residues 347 - 355, but this antibody clearly benefits from the addition of residues from the range 135 - 143 to the peptide. The mimics that are produced are still suboptimal, which is reflected in the fact that 1000-fold higher concentrations of antibody are needed to obtain similar signal intensities as are recorded for rabbit Ab 137604, further demonstrating the additional requirements posed on the binding substrate.

This does not have implications for the affinity towards the target protein, which is to be determined by quantitative methods (e.g. Biacore). In fact exquisite selectivity hallmarks antibodies that can be used *in vivo* without causing side effects.

**Table 12: Epitopes of the antibodies in this study**

| **Antibody** | **Epitope** | **Most important residues** | **Conformation sensitive** |
|---|---|---|---|
| **Rabbit Anti Glypican 1/GPC1 antibody (ab137604)** | ₃₄₈VNPQGPGPEE_{3S7} **(SEQ ID NO: 3)** | P353, G354, E356 | N |
| **Goat Anti-Glypican 1 (GPC1) (AA 24-530)** | ₃₄₉NPQGPGPEE_{3S7} **(SEQ ID NO: 4)** | G352, G354, E356 | ? |
| **MIL38-AM4** | ₃₄₇KVNPQGPGP₃₅₅ **(SEQ ID NO: 6)** | V348, Q351, G352, P353 | Y |

Based on the results presented above, **Table 13** below represents a summary of substitutions tolerated in the epitope sequences analysed.

**Table 13: Summary of tolerated substitutions in epitope sequences**

| **Residue No. in SEQ ID NO: 14** | **347** | **348** | **349** | **350** | **351** | **352** | **353** | **354** | **355** | **356 E** | **357 E** | **358** |
|---|---|---|---|---|---|---|---|---|---|---|---|---|
| | **K** | **V** | **N** | **P** | **Q** | **G** | **P** | **G** | **P** | | | **K** |
| | | | | | | | | | | | | |
| **Tolerated substitutions in MIL38-AM4 Ab epitope** | WRLYF | X | HPD | RKWSHN | X | X | X | DENQKRA | MAIKRQSTY | ✔ | ✔ | ✔ |
| | | | | | | | | | | | | |
| **Tolerated substitutions in Goat Anti-Glypican 1 (GPC1) (AA 24-530) Ab epitope** | ✔ | ✔ | H | ✔ | NMTSR | X | A | X | NOT RNQVIL | X | ✔ | ✔ |
| | | | | | | | | | | | | |
| **Tolerated substitutions in Rabbit Anti Glypican 1/GPC1 antibody (ab137604) epitope** | ✔ | ✔ | ✔ | ✔ | YAEVMFLITR | ASTHWYFM | X | X | ✔ | X | QDFHM | ✔ |

| | | | | | | | | | | | | |
|---|---|---|---|---|---|---|---|---|---|---|---|---|
| ✔ Means that any amino acid substitution can be tolerated at this position X Means that no amino acid substitution can be tolerated at this position *A (ala), R (arg), N (asn), (asp), B (asx), C (cys), E (glu), Q (gln), Z (glx), G (gly), H (his), I (ile), L (leu), K (lys), M (met), F (phe), P (pro), S (ser), T (thr), W (trp), Y (tyr), or V (val)* | | | | | | | | | | | | |

### Example 4. MIL38 and anti-glypican-1 (anti-GPC-1) antibodies show overlapping reactivity on 2D western blots.

Rabbit anti-GPC-1 antibody ab137604 showed reactivity with the glypican-1 core protein at a molecular weight of approximately 60 kDa - the same molecular weight as detected by MIL38. To confirm that MIL38 recognized glypican-1, prostate cancer DU-145 MPEK extracts were subjected to 2D electrophoresis and western blotting.

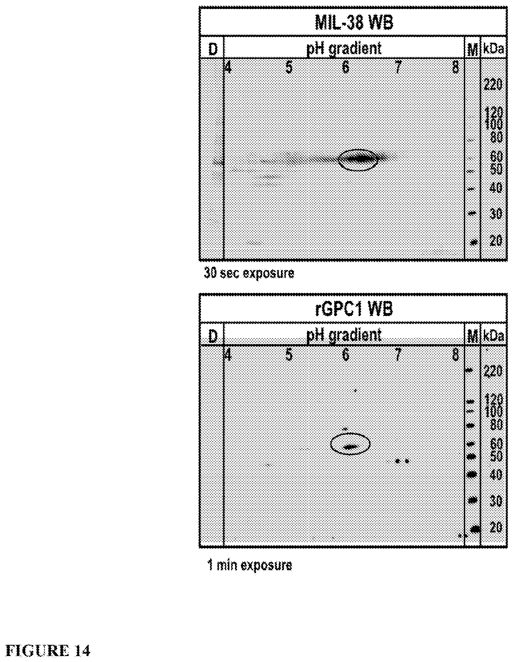

Membrane protein extracts (MPEK) of DU-145 prostate cancer cells were separated on 2D gel (pI gradient-horizontal, and molecular mass vertical). Western blots using MIL38 antibody and commercial rGPC-1 rabbit polyclonal antibodies show overlapping reactivity marking a 60Kd protein (circled in **Figure 14**). **Lane D** (**Figure 14**) is a one dimension separation for DU-145 extracts as a control. **Lane M** (**Figure 14**) is a one dimension separation lane for molecular size markers as controls.

As shown in **Figure 14****,** MIL38 antibody and the anti-GPC-1 antibodies showed overlapping reactivity detecting a protein with 60 kDa molecular weight and isoelectric points ranging from 5 to 7.

### Example 5. MIL38 is detected in anti-GPC-1 immunoprecipitates and vice versa.

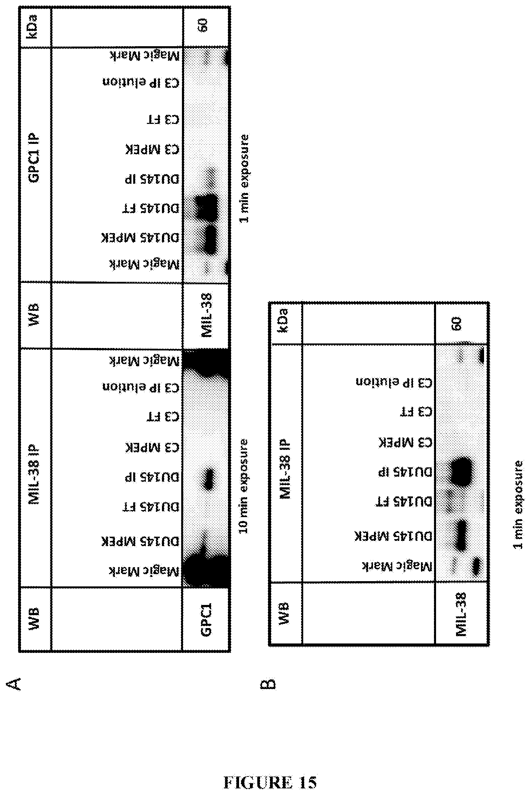

MIL38 or rabbit anti-GPC-1 antibodies were used to immunoprecipitate their respective antigens from DU-145 or C3 (MIL38 negative) MPEK extracts. The immunoprecipitates (IPs) were western blotted with either MIL38 or anti-GPC-1 antibody **(****Figure 15****).**

A 60kDa GPC-1 reactive band was detected in MIL38 IPs blotted with anti-GPC-1, while a 60 kDa MIL38 reactive band was detected in anti-GPC-1 IPs blotted with MIL38. No reactivity was detected with the secondary only controls. Furthermore, immunoprecipitating with MIL38 antibody resulted in almost complete depletion of both MIL38 and anti-GPC-1 antigens, strongly suggesting that the MIL38 antigen is glypican-1.

MIL38 and rabbit anti-GPC-1 antibodies were each used to immunoprecipitate their antigens from DU-145 prostate cancer or C3 (MIL38 negative) cell membrane protein extracts. Shown are the western blots of the immunoprecipitations detected with either MIL38 or anti-GPC-1 antibody. **Figure 15A** depicts GPC1 detection of MIL38 immunoprecipitates (left) and MIL38 detection of GPC1 immunoprecipitates (right). **Figure 15B** depicts MIL38 detection of MIL38 immunoprecipitates as a control. Lanes are: Magic Mark-commercial protein marker as control; DU145 MPEK- prostate cancer membrane protein extract (not immunoprecipitated); DU145 FT- prostate cancer flow through from immune precipitation; DU145 IP- immunoprecipitate using antibody; C3 MPEK- (MIL38 negative) control membrane protein extract (not immunoprecipitated); C3 FT- flow through from immune precipitation; C3 IP elution- (MIL38 negative) immunoprecipitate using antibody. MIL38 can detect the immunoprecipitate from rGPC1 antibody and vice versa. MIL38 can also bind to all controls including DU145 MPEK and to IP conducted by MIL38.

### Example 6. GPC-1 can be detected by MIL38 in prostate cancer plasma samples and in membrane extracts from prostate cancer patients.

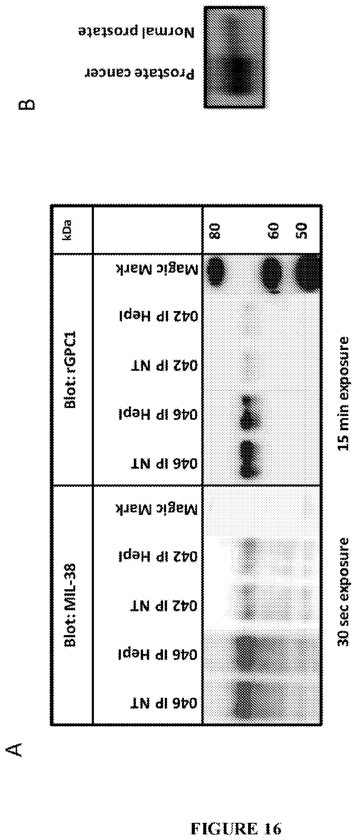

Plasma samples from one normal patient (042) and one prostate cancer patient (046) were immunoprecipitated with MIL38 antibody and the IP sample western blotted with MIL38 and anti-GPC-1 antibodies (**Figure 16A**).

Both antibodies detected specific bands of approx. 70kDa in both plasma samples. The signals were markedly higher (darker bands) for both MIL38 and anti-GPC-1 antibodies in the prostate cancer patient plasma compared to the normal patient plasma, suggesting that this soluble form of glypican-1 may be elevated in prostate cancer patients.

To determine if MIL38 antigen could be detected in membrane protein extracts from normal prostate and prostate cancer, one sample of each was obtained from Novus Bio. Equivalent amounts of protein were western blotted using MIL38 antibody **(****Figure 16B****).** The prostate cancer extract demonstrated much higher expression of the MIL38 antigen than the normal prostate sample.

### Example 7. Detection of MIL38 antigen in patient urine.

MIL38 can detect cells in the urine of prostate cancer patients. To test the sensitivity and specificity of this detection method, 125 age-matched urine samples were obtained. Cells were spun down from the urine and analyzed by the MIL38 indirect immunofluorescence assay. A total of 47 healthy controls, 37 benign prostatic hypertrophy (BPH) and 41 biopsy-confirmed prostate cancers were analyzed.

The MIL38 immunofluorescence assay (IFA) test demonstrated a sensitivity of 71% and a specificity of 73% in identifying prostate cancers within the cohort. The test showed 71% sensitivity and 76% specificity in identifying prostate cancers compared to BPH patients **(Table 14).**

**Table 14: Sensitivity and specificity calculations of prostate cancer detection in patient urine.**

| **Sensitivity and Specificity Calculations** | |
|---|---|
| True Positive | False Positive |
| 29 | 12 |
| False Negative | True Negative |
| 23 | 61 |

| **Sensitivity and Specificity Calculations for BPH only** | |
|---|---|
| True Positive | False Positive |
| 29 | 12 |
| False Negative | True Negative |
| 9 | 28 |

## Claims

1. An isolated epitope or epitope segment for an anti-glypican 1 (GPC-1) antibody located within a portion of the GPC-1 flexible loop defined by an amino acid sequence KVNPQGPGPEEK **(SEQ ID NO: 1),** wherein the isolated epitope or epitope segment consists of:
(i) KVNPQGPGPEEK **(SEQ ID NO: 1);**
(ii) a fragment of KVNPQGPGPEEK **(SEQ ID NO: 1)** consisting of VNPQGPGPEEK **(SEQ ID NO: 2),** VNPQGPGPEE **(SEQ ID NO: 3),** NPQGPGPEE **(SEQ ID NO: 4),** KVNPQGPGPE **(SEQ ID NO: 5)** or KVNPQGPGP **(SEQ ID NO: 6);**
(iii) a variant of **SEQ ID NO: 3** with a substitution at any one of:
position 1, wherein V (val) is substituted with any other amino acid,
position 2, wherein N (asn) is substituted with any other amino acid,
position 3, wherein P (pro) is substituted with any other amino acid,
position 4, wherein Q (gln) is substituted with any one of Y (tyr), A (ala), E (glu), V (val), M (met), F (phe), L (leu), I (ile), T (thr), or R (arg),
position 5, wherein G (gly) is substituted with A (ala), S (ser), T (thr), H (his), W (trp), Y (tyr), F (phe), or M (met),
position 8, wherein P (pro) is substituted with any other amino acid, or
position 10, wherein E (glu) is substituted with Q (gln), D (asp), F (phe), H (his) or M (met),
(iv) a variant of **SEQ ID NO: 4** with a substitution at any one of:
position 1, wherein N (asn) is substituted with H (his),
position 2, wherein P (pro) is substituted with any other amino acid,
position 3, wherein Q (gln) is substituted with any one of N (asn), M (met), T (thr), S (ser), or R (arg),
position 5, wherein P (pro) is substituted with A (ala),
position 7, wherein P (pro) is substituted with any one of A (ala), D (asp), C (cys), E (glu), Z (glx), G (gly), H (his), K (lys), M (met), F (phe), P (pro), S (ser), T (thr), W (trp), or Y (tyr), or
position 9, wherein E (glu) is substituted with any other amino acid; or
(v) a variant of **SEQ ID NO: 5** or **SEQ ID NO: 6** with a substitution only at any one of:
position 1, wherein K (lys) is substituted with any one of W (trp), R (arg), L (lys), Y (tyr) or F (phe);
position 3, wherein N (asn) is substituted with any one of H (his), P (pro) or D (asp);
position 4, wherein P (pro) is substituted with any one of R (arg), K (lys), W (trp), S (ser), H (his) or N (asn);
position 8, wherein G (gly) is substituted with any one of D (asp), E (glu), N (asn), Q (gln), K (lys), R (arg) or A (ala); or
position 9, wherein P (pro) is substituted with any one of M (met), A (ala), I (ile), K (lys), R (arg), Q (gln), S (ser), T (thr), or Y (tyr).

2. An isolated epitope consisting of a first segment according to claim 1 and a second segment, wherein:
(i) the first segment is: the fragment of KVNPQGPGPEEK **(SEQ ID NO:** 1) consisting of VNPQGPGPEEK **(SEQ ID NO: 2),** KVNPQGPGPE **(SEQ ID NO: 5)** or KVNPQGPGP **(SEQ ID NO: 6);** the variant of KVNPQGPGPE **(SEQ ID NO: 5);** or the variant of KVNPQGPGP **(SEQ ID NO: 6);** and
(ii) the second segment consists of an amino acid sequence TQNARA **(SEQ ID NO: 8).**

3. The epitope according to claim 2, wherein the second segment consists of an amino acid sequence TQNARAFRD **(SEQ ID NO: 7).**

4. An isolated epitope consisting of a first segment according to claim 1 and a second segment, wherein:
(i) the first segment is: the fragment of KVNPQGPGPEEK **(SEQ ID NO: 1)** consisting of NPQGPGPEE **(SEQ ID NO: 4);** or the variant of NPQGPGPEE **(SEQ ID NO: 4);** and
(ii) the second segment consists of an amino acid sequence ALSTASDDR **(SEQ ID NO: 9).**

5. An isolated epitope consisting of a first segment according to claim 1 and a second segment, wherein:
(i) the first segment is: the fragment of KVNPQGPGPEEK **(SEQ ID NO: 1)** consisting of VNPQGPGPEE **(SEQ ID NO: 3);** and
(ii) the epitope second segment consists of:
an amino acid sequence PRERPP **(SEQ ID NO: 10)** or
an amino acid sequence QDASDDGSGS **(SEQ ID NO: 11**).

6. An isolated epitope consisting of a first segment according to claim 1, a second segment, and a third segment wherein:
(i) the first segment is: the fragment of KVNPQGPGPEEK **(SEQ ID NO: 1)** consisting of VNPQGPGPEE **(SEQ ID NO: 3);** and
(ii) the second segment consists of an amino acid sequence PRERPP **(SEQ ID NO: 10),** and the third segment consists of an amino acid sequence QDASDDGSGS **(SEQ ID NO: 11**).

7. An isolated epitope comprising the epitope segment according to claim 1, and consisting of the amino acid sequence CGELYTQNARAFRDLCGNPKVNPQGPGPEEKRRRGC **(SEQ ID NO: 12).**

8. A nucleic acid encoding the epitope according to any one of claims 1 to 7.

9. A method for detecting prostate cancer in a subject, the method comprising detecting the presence of an epitope according to any one of claims 1 to 7 in a biological sample from a subject, and determining that the subject has prostate cancer or an increased likelihood of developing prostate cancer based on amount of the epitope detected in the sample.

10. The method according to claim 9, wherein detecting the presence of the epitope in the sample comprises contacting the sample with a binding entity capable of specifically binding to an epitope according to any one of claims 1 to 7.

11. The method according to claim 10, wherein the binding entity is a population of antibodies.

12. The method according to claim 11, wherein the population of antibodies comprises any one or more of: MIL38 antibody (CBA20140026), rabbit anti-GPC-1 polyclonal antibody (ab137604, abcam), mouse anti-glypican monoclonal antibody 2600 clone 4D1 (Millipore), or goat anti-glypican 1 antibody (AA 24-530).

13. A fusion protein comprising the epitope according to any one of claims 1 to 7.

14. Use of the epitope according to any one of claims 1 to 7, or the fusion protein according to claim 13, as a positive control element in a method for detecting GPC-1.

## Patentansprüche

1. Isoliertes Epitop oder Epitopsegment für einen Anti-Glypican-1(GPC-1)-Antikörper, der innerhalb eines Abschnitts der flexiblen GPC-1-Schleife angeordnet ist, definiert durch eine Aminosäuresequenz KVNPQGPGPEEK (SEQ ID NO: 1), wobei das isolierte Epitop oder Epitopsegment aus Folgendem besteht:
(i) KVNPQGPGPEEK (SEQ ID NO: 1);
(ii) einem Fragment von KVNPQGPGPEEK (SEQ ID NO: 1), bestehend aus VNPQGPGPEEK (SEQ ID NO: 2), VNPQGPGPEE (SEQ ID NO: 3), NPQGPGPEE (SEQ ID NO: 4), KVNPQGPGPE (SEQ ID NO: 5) oder KVNPQGPGP (SEQ ID NO: 6);
(iii) einer Variante von SEQ ID NO: 3 mit einer Substitution entweder an:
Position 1, wobei V (Val) durch irgendeine andere Aminosäure ersetzt ist,
Position 2, wobei N (Asn) durch irgendeine andere Aminosäure ersetzt ist,
Position 3, wobei P (Pro) durch irgendeine andere Aminosäure ersetzt ist,
Position 4, wobei Q (Gln) durch eines aus Y (Tyr), A (Ala), E (Glu), V (Val), M (Met), F (Phe), L (Leu), I (Ile), T(Thr) oder R (Arg) ersetzt ist,
Position 5, wobei G (Gly) durch A (Ala), S (Ser), T (Thr), H (His), W (Trp), Y (Tyr), F (Phe) oder M (Met) ersetzt ist,
Position 8, wobei P (Pro) durch irgendeine andere Aminosäure ersetzt ist, oder
Position 10, wobei E (Glu) durch Q (Gln), D (Asp), F (Phe), H (His) oder M (Met) ersetzt ist,
(iv) einer Variante von SEQ ID NO: 4 mit einer Substitution entweder an:
Position 1, wobei N (Asn) durch H (His) ersetzt ist,
Position 2, wobei P (Pro) durch irgendeine andere Aminosäure ersetzt ist,
Position 3, wobei Q (Gln) durch eines aus N (Asn), M Met), T (Thr), S (Ser) oder R (Arg) ersetzt ist,
Position 5, wobei P (Pro) durch A (Ala) ersetzt ist,
Position 7, wobei P (Pro) durch eines aus A (Ala), D (Asp), C (Cys), E (Glu), Z (Glx), G (Gly), H (His), K (Lys), M (Met), F (Phe), P (Pro), S (Ser), T (Thr), W (Trp) oder Y (Tyr) ersetzt ist, oder
Position 9, wobei E (Glu) durch irgendeine andere Aminosäure ersetzt ist; oder
(v) einer Variante von SEQ ID NO: 5 oder SEQ ID NO: 6 mit einer Substitution lediglich entweder an
Position 1, wobei K (Lys) durch eines aus W (Trp), R (Arg), L (Lys), Y (Tyr) oder F (Phe) ersetzt ist;
Position 3, wobei N (Asn) durch eines aus H (His), P (Pro) oder D (Asp) ersetzt ist,
Position 4, wobei P (Pro) durch eines aus R (Arg), K (Lys), W (Trp), S (Ser), H (His) oder N (Asn) ersetzt ist;
Position 8, wobei G (Gly) durch eines aus D (Asp), E (Glu), N (Asn), Q (Gln), K (Lys), R (Arg) oder A (Ala) ersetzt ist; oder
Position 9, wobei P (Pro) durch eines aus M (Met), A (Ala), I (Ile), K (Lys), R (Arg), Q (Gln), S (Ser), T (Thr) oder Y (Tyr) ersetzt ist.

2. Isoliertes Epitop, bestehend aus einem ersten Segment nach Anspruch 1 und einem zweiten Segment, wobei:
(i) das erste Segment Folgendes ist: das Fragment von KVNPQGPGPEEK (SEQ ID NO: 1), bestehend aus VNPQGPGPEEK (SEQ ID NO: 2), KVNPQGPGPE (SEQ ID NO: 5) oder KVNPQGPGP (SEQ ID NO: 6); die Variante von KVNPQGPGPE (SEQ ID NO: 5) oder die Variante von KVNPQGPGP (SEQ ID NO: 6); und
(ii) das zweite Segment aus einer Aminosäuresequenz TQNARA (SEQ ID NO: 8) besteht.

3. Epitop nach Anspruch 2, wobei das zweite Segment aus einer Aminosäuresequenz TQNARAFRD (SEQ ID NO: 7) besteht.

4. Isoliertes Epitop, bestehend aus einem ersten Segment nach Anspruch 1 und einem zweiten Segment, wobei:
(i) das erste Segment Folgendes ist: das Fragment von KVNPQGPGPEEK (SEQ ID NO: 1), bestehend aus NPQGPGPEE (SEQ ID NO: 4); oder die Variante von NPQGPGPEE (SEQ ID NO: 4); und
(ii) das zweite Segment aus einer Aminosäuresequenz ALSTASDDR (SEQ ID NO: 9) besteht.

5. Isoliertes Epitop, bestehend aus einem ersten Segment nach Anspruch 1 und einem zweiten Segment, wobei:
(i) das erste Segment Folgendes ist: das Fragment von KVNPQGPGPEEK (SEQ ID NO: 1), bestehend aus VNPQGPGPEE (SEQ ID NO: 3); und
(ii) das zweite Epitopsegment aus Folgendem besteht:
einer Aminosäuresequenz PRERPP (SEQ ID NO: 10) oder
einer Aminosäuresequenz QDASDDGSGS (SEQ ID NO: 11).

6. Isoliertes Epitop, bestehend aus einem ersten Segment nach Anspruch 1, einem zweiten Segment und einem dritten Segment, wobei:
(i) das erste Segment Folgendes ist: das Fragment von KVNPQGPGPEEK (SEQ ID NO: 1), bestehend aus VNPQGPGPEE (SEQ ID NO: 3); und
(ii) das zweite Segment aus einer Aminosäuresequenz PRERPP (SEQ ID NO: 10) besteht und das dritte Segment aus einer Aminosäuresequenz QDASDDGSGS (SEQ ID NO: 11) besteht.

7. Isoliertes Epitop, umfassend das Epitopsegment nach Anspruch 1 und bestehend aus der Aminosäuresequenz
CGELYTQNARAFRDLCGNPKVNPQGPGPEEKRRRGC (SEQ ID NO: 12).

8. Nukleinsäure, welche das Epitop nach einem der Ansprüche 1 bis 7 codiert.

9. Verfahren zum Erkennen von Prostatakrebs bei einem Patienten, wobei das Verfahren Erkennen des Vorliegens eines Epitops nach einem der Ansprüche 1 bis 7 in einer biologischen Probe von dem Patienten und Bestimmen, dass der Patient an Prostatakrebs erkrankt ist oder eine erhöhte Wahrscheinlichkeit aufweist, Prostatakrebs zu entwickeln, basierend auf der Menge des in der Probe erkannten Epitops umfasst.

10. Verfahren nach Anspruch 9, wobei das Erkennen des Vorliegens des Epitops in der Probe In-Kontakt-Bringen der Probe mit einer Bindungseinheit umfasst, die in der Lage ist, spezifisch an ein Epitop nach einem der Ansprüche 1 bis 7 zu binden.

11. Verfahren nach Anspruch 10, wobei die Bindungseinheit eine Population von Antikörpern ist.

12. Verfahren nach Anspruch 11, wobei die Population von Antikörpern einen beliebigen oder mehrere beliebige aus Folgendem umfasst: MIL38-Antikörper (CBA20140026), polyklonalem Kaninchen-Anti-GPC-1-Antikörper (ab137604, Abcam), monoklonalem Maus-Anti-Glypican-Antikörper 2600 Klon 4D1 (Millipore) oder Ziegen-Anti-Glypican-1-Antikörper (AA 24-530).

13. Fusionsprotein, umfassend das Epitop nach einem der Ansprüche 1 bis 7.

14. Verwendung des Epitops nach einem der Ansprüche 1 bis 7 oder des Fusionsproteins nach Anspruch 13 als ein positives Kontrollelement in einem Verfahren zum Erkennen von GPC-1.

## Revendications

1. Épitope isolé ou segment d'épitope pour un anticorps dirigé contre le glypicane 1 (GPC-1) situé à l'intérieur de la boucle flexible de GPC-1 définie par une séquence d'acides aminés KVNPQGPGPEEK (SEQ ID N° : 1), l'épitope isolé ou le segment d'épitope étant constitué par :
(i) KVNPQGPGPEEK (SEQ ID N° : 1) ;
(ii) un fragment de KVNPQGPGPEEK (SEQ ID N° : 1) constitué par VNPQGPGPEEK (SEQ ID N° : 2), VNPQGPGPEE (SEQ ID N° : 3), NPQGPGPEE (SEQ ID N° : 4), KVNPQGPGPE (SEQ ID N° : 5) ou KVNPQGPGP (SEQ ID N° : 6);
(iii) une variante de la SEQ ID N° : 3 présentant une substitution au niveau de l'une quelconque parmi :
la position 1, où V (val) est remplacé par n'importe quel autre acide aminé,
la position 2, où N (asn) est remplacé par n'importe quel autre acide aminé,
la position 3, où P (pro) est remplacé par n'importe quel autre acide aminé,
la position 4, où Q (gln) est remplacé par l'un quelconque parmi Y (tyr), A (ala), E (glu), V (val), M (met), F (phe), L (leu), I (ile), T (thr), ou R (arg),
la position 5, où G (gly) est remplacé par A (ala), S (ser), T (thr), H (his), W (trp), Y (tyr), F (phe), ou M (met),
la position 8, où P (pro) est remplacé par n'importe quel autre acide aminé, ou
la position 10, où E (glu) est remplacé par Q (gln), D (asp), F (phe), H (his) ou M (met),
(iv) une variante de la SEQ ID N° : 4 présentant une substitution au niveau de l'une quelconque parmi :
la position 1, où N (asn) est remplacé par H (his),
la position 2, où P (pro) est remplacé par n'importe quel autre acide aminé,
la position 3, où Q (gln) est remplacé par l'un quelconque parmi N (asn), M (met), T (thr), S (ser), ou R (arg),
la position 5, où P (pro) est remplacé par A (ala),
la position 7, où P (pro) est remplacé par l'un quelconque parmi A (ala), D (asp), C (cys), E (glu), Z (glx), G (gly), H (his), K (lys), M (met), F (phe), P (pro), S (ser), T (thr), W (trp), ou Y (tyr), ou
la position 9, où E (glu) est remplacé par n'importe quel autre acide aminé; ou
(v) une variante de la SEQ ID N° : 5 ou de la SEQ ID N° : 6 présentant une substitution au niveau de l'une quelconque parmi :
la position 1, où K (lys) est remplacé par l'un quelconque parmi W (trp), R (arg), L (lys), Y (tyr) ou F (phe) ;
la position 3, où N (asn) est remplacé par l'un quelconque parmi H (his), P (pro) ou D (asp) ;
la position 4, où P (pro) est remplacé par l'un quelconque parmi R (arg), K (lys), W (trp), S (ser), H (his) ou N (asn) ;
la position 8, où G (gly) est remplacé par l'un quelconque parmi D (asp), E (glu), N (asn), Q (gln), K (lys), R (arg) ou A (ala) ; ou
la position 9, où P (pro) est remplacé par l'un quelconque parmi M (met), A (ala), I (ile), K (lys), R (arg), Q (gln), S (ser), T (thr), ou Y (tyr).

2. Épitope isolé constitué d'un premier segment selon la revendication 1 et d'un deuxième segment, où :
(i) le premier segment est : le fragment de KVNPQGPGPEEK (SEQ ID N° : 1) constitué par VNPQGPGPEEK (SEQ ID N° : 2), KVNPQGPGPE (SEQ ID N° : 5) ou KVNPQGPGP (SEQ ID N° : 6) ; la variante de KVNPQGPGPE (SEQ ID N° : 5) ; ou la variante de KVNPQGPGP (SEQ ID N° : 6) ; et
(ii) le deuxième segment est constitué par une séquence d'acides aminés TQNARA (SEQ ID N° : 8).

3. Épitope selon la revendication 2, dans lequel le deuxième segment est constitué par une séquence d'acides aminés TQNARAFRD (SEQ ID N° : 7).

4. Épitope isolé constitué d'un premier segment selon la revendication 1 et d'un deuxième segment, où :
(i) le premier segment est : le fragment de KVNPQGPGPEEK (SEQ ID N° : 1) constitué par NPQGPGPEE (SEQ ID N° : 4) ; ou la variante de NPQGPGPEE (SEQ ID N° : 4) ; et
(ii) le deuxième segment est constitué par une séquence d'acides aminés ALSTASDDR (SEQ ID N° : 9).

5. Épitope isolé constitué d'un premier segment selon la revendication 1 et d'un deuxième segment, où :
(i) le premier segment est : le fragment de KVNPQGPGPEEK (SEQ ID N° : 1) constitué par VNPQGPGPEE (SEQ ID N° : 3) ; et
(ii) le deuxième segment d'épitope est constitué par :
une séquence d'acides aminés PRERPP (SEQ ID N° : 10) ou
une séquence d'acides aminés QDASDDGSGS (SEQ ID N° : 11).

6. Épitope isolé constitué d'un premier segment selon la revendication 1, d'un deuxième segment et d'un troisième segment, où :
(i) le premier segment est : le fragment de KVNPQGPGPEEK (SEQ ID N° : 1) constitué par VNPQGPGPEE (SEQ ID N° : 3) ; et
(ii) le deuxième segment est constitué par une séquence d'acides aminés PRERPP (SEQ ID N° : 10), et le troisième segment est constitué par une séquence d'acides aminés QDASDDGSGS (SEQ ID N° : 11).

7. Épitope isolé comprenant le segment d'épitope selon la revendication 1, et constitué par la séquence d'acides aminés
CGELYTQNARAFRDLCGNPKVNPQGPGPEEKRRRGC (SEQ ID N° : 12).

8. Acide nucléique codant pour l'épitope selon l'une quelconque des revendications 1 à 7.

9. Procédé de détection d'un cancer de la prostate chez un sujet, le procédé comprenant la détection de la présence d'un épitope selon l'une quelconque des revendication 1 à 7 dans un échantillon biologique provenant d'un sujet, et la détermination que le sujet présente un cancer de la prostate ou une probabilité accrue de développer un cancer de la prostate sur la base de la quantité d'épitope détectée dans l'échantillon.

10. Procédé selon la revendication 9, dans lequel la détection de la présence de l'épitope dans l'échantillon comprend la mise en contact de l'échantillon avec une entité de liaison apte à se lier spécifiquement à un épitope selon l'une quelconque des revendications 1 à 7.

11. Procédé selon la revendication 10, dans lequel l'entité de liaison est une population d'anticorps.

12. Procédé selon la revendication 11, dans lequel la population d'anticorps comprend un ou plusieurs parmi : un anticorps MIL38 (CBA20140026), un anticorps polyclonal de lapin dirigé contre GPC-1 (ab137604, abcam), un clone 2600 4D1 (Millipore) d'anticorps monoclonal de souris dirigé contre le glypicane, ou un anticorps de chèvre dirigé contre le glypicane 1 (AA 24-530).

13. Protéine de fusion comprenant l'épitope selon l'une quelconque des revendications 1 à 7.

14. Utilisation de l'épitope selon l'une quelconque des revendications 1 à 7, ou de la protéine de fusion selon la revendication 13, comme élément de contrôle positif dans un procédé de détection de GPC-1.


<110> Minomic International Ltd
<120> Glypican Epitopes and Uses Thereof
<130> P098197C
<160> 22
<170> PatentIn version 3.5
<210> 1
   <211> 12
   <212> PRT
   <213> Homo sapiens
<400> 1
   Lys Val Asn Pro Gln Gly Pro Gly Pro Glu Glu Lys
   1               5                   10
<210> 2
   <211> 11
   <212> PRT
   <213> Homo sapiens
<400> 2
   Val Asn Pro Gln Gly Pro Gly Pro Glu Glu Lys
   1               5                   10
<210> 3
   <211> 10
   <212> PRT
   <213> Homo sapiens
<400> 3
   Val Asn Pro Gln Gly Pro Gly Pro Glu Glu
   1               5                   10
<210> 4
   <211> 9
   <212> PRT
   <213> Homo sapiens
<400> 4
   Asn Pro Gln Gly Pro Gly Pro Glu Glu
   1 5
<210> 5
   <211> 10
   <212> PRT
   <213> Homo sapiens
<400> 5
   Lys Val Asn Pro Gln Gly Pro Gly Pro Glu
   1 5 10
<210> 6
   <211> 9
   <212> PRT
   <213> Homo sapiens
<400> 6
   Lys Val Asn Pro Gln Gly Pro Gly Pro
   1 5
<210> 7
   <211> 9
   <212> PRT
   <213> Homo sapiens
<400> 7
   Thr Gln Asn Ala Arg Ala Phe Arg Asp
   1 5
<210> 8
   <211> 6
   <212> PRT
   <213> Homo sapiens
<400> 8
   Thr Gln Asn Ala Arg Ala
   1 5
<210> 9
   <211> 9
   <212> PRT
   <213> Homo sapiens
<400> 9
   Ala Leu Ser Thr Ala Ser Asp Asp Arg
   1 5
<210> 10
   <211> 6
   <212> PRT
   <213> Homo sapiens
<400> 10
   Pro Arg Glu Arg Pro Pro
   1 5
<210> 11
   <211> 10
   <212> PRT
   <213> Homo sapiens
<400> 11
   Gln Asp Ala Ser Asp Asp Gly Ser Gly Ser
   1 5 10
<210> 12
   <211> 36
   <212> PRT
   <213> Homo sapiens
<400> 12
   Cys Gly Glu Leu Tyr Thr Gln Asn Ala Arg Ala Phe Arg Asp Leu Cys
   1 5 10 15
   Gly Asn Pro Lys Val Asn Pro Gln Gly Pro Gly Pro Glu Glu Lys Arg
   20 25 30
   Arg Arg Gly Cys
   35
<210> 13
   <211> 11
   <212> PRT
   <213> Homo sapiens
<400> 13
   Leu Gly Pro Glu Cys Ser Arg Ala Val Met Lys
   1 5 10
<210> 14
   <211> 558
   <212> PHT
   <213> Homo sapiens
<400> 14
   Met Glu Leu Arg Ala Arg Gly Trp Trp Leu Leu Cys Ala Ala Ala Ala
   1               5                   10                  15
   Leu Val Ala Cys Ala Arg Gly Asp Pro Ala Ser Lys Ser Arg Ser Cys
               20                  25                  30
   Gly Glu Val Arg Gln Ile Tyr Gly Ala Lys Gly Phe Ser Leu Ser Asp
           35                  40                  45
   Val Pro Gln Ala Glu Ile Ser Gly Glu His Leu Arg Ile Cys Pro Gln
       50                  55                  60
   Gly Tyr Thr Cys Cys Thr Ser Glu Met Glu Glu Asn Leu Ala Asn Arg
   65                  70                  75                  80
   Ser His Ala Glu Leu Glu Thr Ala Leu Arg Asp Ser Ser Arg Val Leu
                   85                  90                  95
   Gln Ala Met Leu Ala Thr Gln Leu Arg Ser Phe Asp Asp His Phe Gln
               100                 105                 110
   His Leu Leu Asn Asp Ser Glu Arg Thr Leu Gln Ala Thr Phe Pro Gly
           115                 120                 125
   Ala Phe Gly Glu Leu Tyr Thr Gln Asn Ala Arg Ala Phe Arg Asp Leu
       130                 135                 140
   Tyr Ser Glu Leu Arg Leu Tyr Tyr Arg Gly Ala Asn Leu His Leu Glu
   145                 150                 155                 160
   Glu Thr Leu Ala Glu Phe Trp Ala Arg Leu Leu Glu Arg Leu Phe Lys
                   165                 170                 175
   Gln Leu His Pro Gln Leu Leu Leu Pro Asp Asp Tyr Leu Asp Cys Leu
               180                 185                 190
   Gly Lys Gln Ala Glu Ala Leu Arg Pro Phe Gly Glu Ala Pro Arg Glu
           195                 200                 205
   Leu Arg Leu Arg Ala Thr Arg Ala Phe Val Ala Ala Arg Ser Phe Val
       210                 215                 220
   Gln Gly Leu Gly Val Ala Ser Asp Val Val Arg Lys Val Ala Gln Val
   225                 230                 235                 240
   Pro Leu Gly Pro Glu Cys Ser Arg Ala Val Met Lys Leu Val Tyr Cys
                   245                 250                 255
   Ala His Cys Leu Gly Val Pro Gly Ala Arg Pro Cys Pro Asp Tyr Cys
               260                 265                 270
   Arg Asn Val Leu Lys Gly Cys Leu Ala Asn Gln Ala Asp Leu Asp Ala
           275                 280                 285
   Glu Trp Arg Asn Leu Leu Asp Ser Met Val Leu Ile Thr Asp Lys Phe
       290                 295                 300
   Trp Gly Thr Ser Gly Val Glu Ser Val Ile Gly Ser Val His Thr Trp
   305                 310                 315                 320
   Leu Ala Glu Ala Ile Asn Ala Leu Gln Asp Asn Arg Asp Thr Leu Thr
                   325                 330                 335
   Ala Lys Val Ile Gln Gly Cys Gly Asn Pro Lys Val Asn Pro Gln Gly
               340                 345                 350
   Pro Gly Pro Glu Glu Lys Arg Arg Arg Gly Lys Leu Ala Pro Arg Glu
           355                 360                 365
   Arg Pro Pro Ser Gly Thr Leu Glu Lys Leu Val Ser Glu Ala Lys Ala
       370                 375                 380
   Gln Leu Arg Asp Val Gln Asp Phe Trp Ile Ser Leu Pro Gly Thr Leu
   385                 390                 395                 400
   Cys Ser Glu Lys Met Ala Leu Ser Thr Ala Ser Asp Asp Arg Cys Trp
                   405                 410                 415
   Asn Gly Met Ala Arg Gly Arg Tyr Leu Pro Glu Val Met Gly Asp Gly
               420                 425                 430
   Leu Ala Asn Gln Ile Asn Asn Pro Glu Val Glu Val Asp Ile Thr Lys
           435                 440                 445
   Pro Asp Met Thr Ile Arg Gln Gln Ile Met Gln Leu Lys Ile Met Thr
       450                 455                 460
   Asn Arg Leu Arg Ser Ala Tyr Asn Gly Asn Asp Val Asp Phe Gln Asp
   465                 470                 475                 480
   Ala Ser Asp Asp Gly Ser Gly Ser Gly Ser Gly Asp Gly Cys Leu Asp
                   485                 490                 495
   Asp Leu Cys Ser Arg Lys Val Ser Arg Lys Ser Ser Ser Ser Arg Thr
               500                 505                 510
   Pro Leu Thr His Ala Leu Pro Gly Leu Ser Glu Gln Glu Gly Gln Lys
           515                 520                 525
   Thr Ser Ala Ala Ser Cys Pro Gln Pro Pro Thr Phe Leu Leu Pro Leu
       530                 535                 540
   Leu Leu Phe Leu Ala Leu Thr Val Ala Arg Pro Arg Trp Arg
   545                 550                 555
<210> 15
   <211> 23
   <212> PRT
   <213> Homo sapiens
<400> 15
   Gly Asn Pro Lys Val Asn Pro Gln Gly Pro Gly Pro Glu Glu Lys Arg
   1               5                   10                  15
   Arg Arg Gly Lys Leu Ala Pro
               20
<210> 16
   <211> 19
   <212> PRT
   <213> Homo sapiens
<400> 16
   Gly Glu Leu Tyr Thr Gln Asn Ala Arg Ala Phe Arg Asp Leu Tyr Ser
   1               5                   10                  15
   Glu Leu Arg
<210> 17
   <211> 17
   <212> PRT
   <213> Artificial Sequence
<220>
   <223> Description of Artificial Sequence: Labeled homo sapiens Glypican-1 sequence
<400> 17
   Arg Asn Pro Lys Val Asn Pro Gln Gly Pro Gly Pro Glu Glu Lys Arg
   1               5                   10                  15
   Arg
<210> 18
   <211> 15
   <212> PRT
   <213> Homo sapiens
<400> 18
   Pro Lys Val Asn Pro Gln Gly Pro Gly Pro Glu Glu Lys Arg Arg
   1               5                   10                  15
<210> 19
   <211> 17
   <212> PRT
   <213> Artificial Sequence
<220>
   <223> Description of Artificial Sequence: Labeled homo sapiens Glypican-1 sequence
<400> 19
   Arg Asn Glu Leu Cys Thr Gln Asn Cys Arg Ala Phe Arg Asp Leu Tyr
   1               5                   10                  15
   Ser
<210> 20
   <211> 238
   <212> PRT
   <213> Mus musculus
<400> 20
   Met Gly Ile Lys Met Glu Ser Gln Thr Gln Val Phe Val Tyr Met Leu
   1               5                   10                  15
   Leu Trp Leu Ser Gly Val Asp Gly Asp Ile Val Met Thr Gln Ser Gln
               20                  25                  30
   Lys Phe Met Ser Thr Ser Ile Gly Asp Arg Val Ser Val Thr Cys Lys
           35                  40                  45
   Ala Ser Gln Asn Val Gly Ser His Val Ala Trp Phe Gln Gln Lys Pro
       50                  55                  60
   Gly Gln Ser Pro Lys Ala Leu Ile Tyr Ser Ala Ser Tyr Arg Tyr Ser
   65                  70                  75                  80
   Gly Val Thr Asp Arg Phe Thr Gly Ser Gly Ser Gly Thr Asp Phe Thr
                   85                  90                  95
   Leu Thr Ile Asn Asn Val Gln Ser Glu Asp Leu Ala Glu Tyr Phe Cys
               100                 105                 110
   Gln Gln Tyr Asn Ser Phe Pro Phe Thr Phe Gly Ser Gly Thr Lys Leu
           115                 120                 125
   Glu Ile Lys Arg Ala Asp Ala Ala Pro Thr Val Ser Ile Phe Pro Pro
       130                 135                 140
   Ser Ser Glu Gln Leu Thr Ser Gly Gly Ala Ser Val Val Cys Phe Leu
   145                 150                 155                 160
   Asn Asn Phe Tyr Pro Lys Asp Ile Asn Val Lys Trp Lys Ile Asp Gly
                   165                 170                 175
   Ser Glu Arg Gln Asn Gly Val Leu Asn Ser Trp Thr Asp Gln Asp Ser
               180                 185                 190
   Lys Asp Ser Thr Tyr Ser Met Ser Ser Thr Leu Thr Leu Thr Lys Asp
           195                 200                 205
   Glu Tyr Glu Arg His Asn Ser Tyr Thr Cys Glu Ala Thr His Lys Thr
       210                 215                 220
   Ser Thr Ser Pro Ile Val Lys Ser Phe Asn Arg Asn Glu Cys
   225                 230                 235
<210> 21
   <211> 12
   <212> PRT
   <213> Homo sapiens
<400> 21
   Thr Gln Asn Ala Arg Ala Phe Arg Asp Leu Tyr Ser
   1               5                   10
<210> 22
   <211> 1739
   <212> PRT
   <213> Homo sapiens
<400> 22